# EUROPÄISCHE PATENTSCHRIFT

(11) **EP 0 530 625 B1**
(45) Veröffentlichungstag und Bekanntmachung des Hinweises auf die Patenterteilung: **25.09.1996**
(21) Anmeldenummer: 92114370.7
(22) Anmeldetag: 22.08.1992
(51) Int. Cl.: B05B 7/14, A61M 13/00

(54) **Austragvorrichtung für fliessfähige Medien**
Fluid dispenser
Distributeur de fluides

(30) Priorität: 27.08.1991 DE 4128295
(43) Veröffentlichungstag der Anmeldung: 10.03.1993
(73) Patentinhaber: Ing. Erich Pfeiffer GmbH, 78315 Radolfzell (DE)
(72) Erfinder: Güntert, Bernhard, W-7701 Mühlhausen-Ehingen (DE); Wolter, Michael, CH-8266 Steckborn (CH); Ritsche, Stefan, W-7760 Radolfzell (DE); Jäger-Waldau, Reinhold, W-7760 Radolfzell (DE); Fuchs, Karl-Heinz, W-7760 Radolfzell (DE)
(74) Vertreter: Patentanwälte Ruff, Beier, Schöndorf und Mütschele

(56) Entgegenhaltungen:
- WO-A-90/07351
- WO-A-91/06333
- FR-A- 2 224 175

## Beschreibung

Die Erfindung betrifft eine Austragvorrichtung nach dem Oberbegriff des Patentanspruches 1. Sie soll insbesondere für schütt- bzw. rieselfähige Medien aus feinen Feststoffpartikeln geeignet sein, die pulver-, puder- oder mehlförmig sein können. Dieses Medium kann in einem aufgewirbelten, trockenen Staubstrom ausgetragen und/oder wenigstens teilweise vor dem Austrag mit einem weiteren, ggf. flüssigen, Medium vermischt werden. Zum Austrag ist eine willkürlich durch die Bedienungsperson in Gang zu setzende Austragbetätigung vorgesehen, durch welche ein Austragförderer so in Betriebsfunktion versetzt wird, daß das Medium oder die Medienmischung durch eine Austragöffnung aus der Austragvorrichtung heraus bzw. ins Freie ausgetragen werden kann, um seiner bestimmungsgemäßen Anwendung zugeführt zu werden.

Rieselfähige Medien können z.B. für medizinische bzw. therapeutische Zwecke, wie zur Inhalation, oder für technische und andere Zwecke so angewandt werden, daß sie in einer möglichst gleichmäßigen, aufgelockerten Verteilung der im wesentlichen homogen vereinzelten Partikel ausgetragen werden. Hierzu kann erfindungsgemäß die in einem Austragvorgang auszutragende Medienmenge für sich und abgeschlossen von weiteren Vorräten des Mediums in eine Vorlageposition gebracht werden, in welcher das zunächst noch in kompaktem Zustand ruhende Medium einem gasförmigen oder anderen Förderstrom ausgesetzt, durch diesen seine Partikel unter Verwirbelung vereinzelt und dann zur Austragöffnung transportiert wird.

Anstatt den Förderstrom durch Saug- bzw. Atemluft oder einen von der Austragvorrichtung gesonderten Generator zu erzeugen, ist zweckmäßig ein mit der Austragvorrichtung baulich vereinter Generator vorgesehen, dessen Grundkörper im wesentlichen starr mit dem Grundkörper der Austragvorrichtung verbunden ist. Der Generator kann z.B. ein mit Treibgas gefülltes und durch manuelles Öffnen eines Ventiles in Betrieb zu nehmendes Druckgefäß oder bevorzugt eine Luftpumpe sein, welche durch manuellen Antrieb ihrer Pumpbewegung Luft ansaugt, komprimiert und in Richtung zur Vorlagekammer fördert.

Besonders vorteilhaft ist es, wenn der Generator etwa achsparallel, achsgleich und/oder axial unmittelbar benachbart zu einem Magazin für das Medium liegt, da dann bei geringen Außenabmessungen der Austragvorrichtung kurze Leitungswege und ein hoher Wirkungsgrad gewährleistet sind. Ferner kann die Austragvorrichtung zur unangebunden frei getragen einhändigen Betätigung ausgebildet sein, bei welcher mit mindestens zwei einander gegenüberliegenden Fingern der Hand des Benutzers der Förderstrom in Gang gesetzt und dadurch der Medienaustrag bewirkt wird.

Aus der WO-A-91/06333 ist eine Austragvorrichtung bekannt, bei welcher mehrere um eine Lager- bzw. Zentralachse verteilte Vorlagekammern achsparallel zu dieser Zentralachse angeordnet sind und daher parallel zur Zentralachse einen ihrer Länge entsprechenden Aufnahmeraum benötigen.

Der Erfindung liegt des weiteren die Aufgabe zugrunde, eine Austragvorrichtung der genannten Art zu schaffen, bei welcher Nachteile bekannter Ausbildungen oder der beschriebenen Art vermieden sind und die insbesondere bei einfachem und kompaktem Aufbau einen feinstverteilten, gleichmäßigen Austrag einzelner Mediendosen gewährleistet.

Erfindungsgemäß sind die Merkmale des Patentanspruches 1 vorgesehen.

Die Vorrichtung kann einen oder mehrere Magazinkörper, eine oder mehrere Generatoren, eine oder mehrere Austragöffnungen, eine oder mehrere gesondert betätigbare Austragbetätigungen bzw. Austragförderer, ein oder mehrere gesonderte Schaltwerke zur Weiterschaltung des jeweiligen Magazinkörpers, ein oder mehrere gesonderte Nachfülleinrichtungen zur Nachfüllung mindestens einer Magazinkammer mit dem Medium, eine oder mehrere Rüttelvorrichtungen zur Auflockerung bzw. Nachförderung des Mediums und andere noch zu beschreibende Anordnungen aufweisen, die an einer Austragvorrichtung hinsichtlich ihrer Ausbildung und/oder Funktion im wesentlichen gleich oder verschieden ausgebildet sein können.

Zweckmäßig ist ein Magazinkörper mit radialen und/oder axialen Magazinkammern versehen, die kanalförmig derart langgestreckt ausgebildet sein können, daß sie praktisch einen mit dem Medium locker verpfropften Zwischenabschnitt des Förderweges bilden. Dieser Zwischenabschnitt wird dann in der Austragstellung der jeweiligen Magazinkammer nur mit einem Ende an einen zuführenden Abschnitt des Förderweges und mit dem davon abgekehrten Ende an einen wegführenden Abschnitt des Förderweges bzw. unmittelbar an die Austragöffnung angeschlossen, so daß das in der Magazinkammer enthaltene Medium ausgestoßen werden kann. Die Begrenzung der Medienkammer kann dabei unmittelbar durch den Magazinkörper oder durch eine Kapsel gebildet sein, welche in eine entsprechende Magazinaufnahme des Magazinkörpers einzusetzen ist.

Um den Magazinkörper zwischen einer Außerbetriebstellung und einer Austragstellung der jeweiligen Medienkammer bewegen zu können, kann er unmittelbar gleichlaufend mit einer von außen zugänglichen Handhabe verbunden sein oder es kann zwischen einer solchen Handhabe und dem Magazinkörper ein Schalttrieb vorgesehen sein, der z.B. eine axiale Bewegung in eine Quer- bzw. Drehbewegung umsetzt, wobei die Weiterschaltung des Kammerkörpers wegabhängig gleichzeitig mit der Betätigung des Generators und/oder auch unabhängig davon durch eine gesonderte Betätigung erfolgen kann. Für die Weiterschaltung kann der Magazinkörper drehbar und/oder axial verschiebbar angeordnet sein, je nachdem, ob die Magazinkammern nur in einem Ringkranz, in Verschieberichtung parallel zueinander und hintereinander oder in mehreren axial zueinander benachbarten Ringkränzen vorgesehen sind.

Anstatt vorgefüllter Vorlage- bzw. Magazinkammern oder zusätzlich hierzu ist es auch möglich, eine Nachfülleinrichtung für die Vorlagekammer bzw. für mindestens eine Magazinkammer vorzusehen. Diese Nachfülleinrichtung kann einen Drehschieber mit einer oder mehreren Magazinkammern aufweisen, welche zur Nachfüllung in Deckung mit einer Übertrittsöffnung eines Speichermagazines und von dort wieder in Deckung mit anschließenden Abschnitten des Förderweges gebracht werden, so daß durch Füllen eines oder mehrerer leer gewordener Speichermagazine die Vorrichtung immer wieder betriebsbereit gemacht werden kann und hierfür lediglich geeignete Nachfüllpackungen erforderlich sind.

Ein Nachfüllschieber für die Vorlagekammer kann auch linear bewegbar und so angeordnet sein, daß er durch eine Querbewegung durch einen Speicherschacht hindurch bewegt wird und dabei Medium durch eine oder mehrere Wandöffnungen dieses Speicherschachtes in die Vorlagekammer drückt. Durch geeignete, federbelastete oder durch Rüttelbewegungen nachlaufende Begrenzung des das Medium aufnehmenden Speicherraumes kann stets dafür gesorgt werden, daß im Bereich des Transportendes des Schiebers lückenlos Medium im Speicherschacht bereitgestellt ist. Es ist aber auch möglich, den Magazinkörper so auszubilden, daß er nur eine einzige auszubringende Dosis des Mediums aufnimmt, die für jede Austraganwendung der Vorrichtung nach deren Öffnen von außen neu einzubringen ist.

Besonders vorteilhaft ist es, wenn der Magazinkörper im Betriebszustand zwar nach außen vollständig umschlossen innerhalb eines Außengehäuses der Vorrichtung angeordnet, jedoch nach zerstörungsfreiem Öffnen des Gehäuses so zugänglich ist, daß er gegen einen frisch gefüllten Magazinkörper ausgewechselt werden kann und/oder seine Magazinkammern frisch gefüllt werden können. Zu diesem Zweck kann der Magazinkörper zerstörungsfrei lösbar mit einem Gehäusedeckel verbunden sein, von dem er in Öffnungsstellung des Deckels abgenommen werden kann. Dieser Deckel kann gleichzeitig die Betätigungshandhabe zum Weiterschalten des Magazinkörpers und ein Lagerglied für mindestens ein Axial- und/oder Radiallager bilden.

Die Magazinkammern des Magazinkörpers können aber auch zur Aufnahme von Medienkapseln ausgebildet sein, welche zweckmäßig derart dünnwandig aus sprödem Material bestehen, daß sie bei Druck-, Ritz- und/oder Stechbelastung wesentlich großflächiger unter Rißbildung zerspringen, splittern bzw. ausbrechen, als es der Eingriffsfläche des Öffnungswerkzeuges entspricht. Dadurch kann das Öffnungswerkzeug während des Austrages im Bereich des Eingriffes mit der Kapsel verbleiben, ohne gemeinsam mit dieser den Förderweg bzw. die in der Kapsel hergestellte Öffnung zu versperren.

Im Förderweg zwischen einem Druckraum des Generators und der Vorlagekammer bzw. der Austragöffnung sowie im Transport- bzw. Förderweg des Mediums bedarf es keinerlei Ventile, so daß die Austragvorrichtung - ggf. bis auf ein Einlaßventil der Luftpumpe - ventilfrei ausgebildet werden kann. Allerdings kann der Magazinkörper mit den Einlaß- und/oder Auslaßenden seiner Magazinkammern entlang von Schließflächen laufen, die diese Enden erst dann nach Art einer Schiebersteuerung freigeben bzw. öffnen, wenn sich die jeweilige Magazinkammer in Austragstellung befindet.

Es ist aber auch denkbar, insbesondere in Strömungsrichtung vor der Vorlagekammer bzw. der in Austragstellung als solche wirkenden Magazinkammer ein Ventil für den Förderstrom vorzusehen, das druck- und/oder wegabhängig dann öffnet, wenn im Druckraum ein vorbestimmter Überdruck erreicht bzw. die Leitungsverbindung des Förderweges durch die Vorlagekammer und die Austragöffnung hindurch hergestellt bzw. freigegeben ist. Dadurch kann das verhältnismäßig hoch vorkomprimierte Fördermedium schlagartig in die Vorlagekammer entspannt werden, so daß das enthaltene Medium schlagartig aufgelockert und feinstverteilt ausgetrieben wird.

Ist eine Luftpumpe als Druckgas-Generator vorgesehen, so ist deren Druck- bzw. Pumpenkammer zweckmäßig durch denselben Gehäuseteil begrenzt, wie ein Gehäuseraum für die Aufnahme der Vorlagekammer bzw. des Magazinkörpers. Dadurch kann dieser hülsen- bzw. mantelförmige Gehäuseteil an einem Ende mit einem diesen Gehäuseraum verschließenden Deckel und am anderen Ende mit einer verschiebbaren Betätigungshandhabe verschlossen sein, welche einen an der Innenfläche dieses Gehäuseteiles laufenden Pumpkolben trägt. Die beiden voneinander abgekehrten Stirnflächen dieser Deckel, die gleichzeitig die Endflächen der Vorrichtung bilden können, sind als Druck- bzw. Betätigungshandhaben für eine ergonomisch günstige Austragbetätigung geeignet.

Es ist aber auch denkbar, daß von den beiden zur Austragbetätigung gegeneinander zu bewegenden Baugruppen eine im wesentlichen vollständig innerhalb der anderen liegt, so daß die Austragvorrichtung über ihre Länge im wesentlichen von einem einteilig durchgehenden Mantel begrenzt ist. Das Außengehäuse der Vorrichtung kann bis auf den pumpenseitigen Verschluß über seine Länge im wesentlichen konstante Außenweite aufweisen oder im Bereich des Überganges zwischen dem Aufnahmeraum und dem Generator bzw. im Bereich des gegenüberliegenden Deckels so in der Außenweite abgesetzt sein, daß es zur Weiterschaltung oder dgl. an zwei Umfangsbereichen zwischen Fingern jeweils sicher gegriffen werden kann.

Die Austragöffnung ist vorteilhaft durch einen frei über den Außenumfang und etwa in der Ebene der Vorlage- bzw. Magazinkammer vorstehenden Austragstutzen gebildet, der vorzugsweise von der Außenseite des Gehäusemantels in seiner Längsrichtung aufgesetzt und durch eine Schnappverbindung gesichert ist. Dadurch kann ein und dieselbe Austragvorrichtung wahlweise mit unterschiedlichen Austragstutzen versehen werden, z.B. einem nasal einzuführenden Austragdorn oder einem bukal einzuführenden, im Querschnitt flachen Mundstück.

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können. Ausführungsbeispiele der Erfindung sind in den Zeichnungen dargestellt und werden im folgenden näher erläutert. In den Zeichnungen zeigen:
- Fig. 1: eine erfindungsgemäße Austragvorrichtung im Axialschnitt,
- Fig. 2: die Austragvorrichtung gemäß Fig. 1 in verkleinerter und verdrehter Ansicht von außen,
- Fig. 3: einen Querschnitt durch Fig. 1,
- Fig. 4: einen Querschnitt durch Fig. 1,
- Fig. 5: einen Ausschnitt als Querschnitt durch Fig. 4,
- Fig. 6: einen Ausschnitt der Fig. 1 in wesentlich vergrößerter Darstellung,
- Fig. 7: ein Detail der Fig. 1 im Querschnitt und vergrößerter Darstellung,
- Fig. 8: ein Detail der Fig. 1 in einer Abwicklung,
- Fig. 9: eine weitere Ausführungsform teilweise im Axialschnitt,
- Fig. 10: eine weitere Ausführungsform im Axialschnitt,
- Fig. 11: ein Ausführungsbeispiel ähnlich Fig. 1,
- Fig. 12: einen Querschnitt durch Fig. 11,
- Fig. 13: die Austragvorrichtung gemäß Fig. 11 in verkleinerter Außenansicht,
- Fig. 14: eine Draufsicht auf Fig. 13,
- Fig. 15: einen Ausschnitt einer weiteren Austragvorrichtung im Axialschnitt,
- Fig. 16: ein Detail der Fig. 15 vergrößert im Querschnitt,
- Fig. 17: eine weitere Ausführungsform in einer Darstellungsform entsprechend Fig. 15 und
- Fig. 18: den Magazinkörper nach Fig. 17 vor dem Einsetzen in die Austragvorrichtung.

Die Austragvorrichtung 1 weist in im wesentlichen achsgleicher Anordnung einen Austragförderer 2 und eine manuell zu bedienende Austragbetätigung 3 sowie einen Druckgas-Generator 4 auf, der Bestandteil des Austragförderers 2 so sein kann, daß im wesentlichen gleichzeitig mit der Betätigung des Generators 4 und durch diese Betätigung Medium gefördert wird. Zur Betätigung sind voneinander abgekehrte Endflächen der Austragvorrichtung 1 als erste und zweite Handhaben 5, 6 vorgesehen, die bei gegenseitiger Annäherung durch Fingerdruck zwischen den Fingern einer Hand den Austrag einer Dosiermenge an Medium sowie ggf. andere Steuervorgänge bewirken und beim Freigeben von selbst wieder in ihre Ausgangslage zurückkehren. Zur Durchführung eines der genannten Steuervorgänge ist des weiteren eine Umfangsfläche als Handhabe 7 ausgebildet, die starr mit der Handhabe 5 verbunden ist.

Die im wesentlichen aus formstabilen Kunststoff-Bauteilen zusammengesetzte Austragvorrichtung 1 weist einen formfesten, hohlen Grundkörper 8 auf, welcher einen größeren Längsabschnitt des Außenmantels eines Gehäuses 9 bildet, dessen Außenform im wesentlichen symmetrisch zu einer Zentralachse 10 vorgesehen ist. Das Gehäuse 9 weist einen über den größten Teil seiner Länge bis an seinen Außenumfang reichenden, annähernd hülsenförmigen Gehäuseteil 12 auf, der über seine gesamte Länge im wesentlichen konstante Innen- und/oder Außenweite hat sowie im wesentlichen den Grundkörper 8 bildet.

An einem Hülsenende ist der Gehäuseteil 12 mit einem im Betriebszustand gegen Akialbewegungen gesicherten, kappenförmigen Deckel 13 etwa gleicher Mantelweite verschlossen, der mit der Außenseite seiner Kappenstirnwand die in Betätigungsrichtung starr mit dem Grundkörper 8 verbundene Handhabe 5 bildet. Durch den Deckel 13 ist ein durch den Innenumfang des Gehäuseteiles 12 begrenzter Gehäuseraum 14 im wesentlichen staubdicht verschlossen, in welchem ein Magazin 15 mit Einzelportionen des auszubringenden Mediums auswechselbar angeordnet ist.

Das Magazin 15 weist einen ringförmig um die Zentralachse 10 angeordneten, zwischen seinem Innen- und seinem Außenumfang ringscheibenförmigen Magazinkörper 16 auf, der aus zwei im wesentlichen gleichen und miteinander z.B. durch flächige Schweißung in der Trennebene verbundenen Bauteilen 17, 18 besteht, die axial aneinander schließen und deren Teilungsebene in der gemeinsamen Mittelebene von Magazinkammern 30 für das Medium liegen.

Der Generator 4 ist im wesentlichen durch eine in der Zentralachse 10 liegende Schubkolben- bzw. Luftpumpe 19 gebildet, deren Pumpenkammer bzw. Druckraum 21 auslaßseitig über einen Förderweg 20 mit einer ins Freie führenden Austragöffnung 11 unter Zwischenschaltung mindestens einer Magazinkammer 30 zu verbinden ist. Im etwa gleichen Abstand zwischen seinen Enden ist der Gehäuseteil 12 mit einer annähernd ringscheibenförmigen Querwand 22 versehen, welche einen Teil der druckdichten Trennung zwischen Gehäuseraum 14 und Druckraum 21 bildet. Im Anschluß an die Querwand 22 bildet die Innenfläche des Mantels des Gehäuseteiles 12 die Kolbenlaufbahn 23 für einen Pumpkolben 25 einer Kolbeneinheit der Luftpumpe 19, so daß diese Kolbenlaufbahn 23 einteilig mit mindestens einer Lagerfläche für den Magazinkörper 16 ausgebildet ist.

Das zugehörige Ende des Gehäuseteiles 12 ist ebenfalls mit einem kappenförmigen, jedoch gegenüber dem Grundkörper 8 axial verschiebbaren Deckel 24 verschlossen, der in das Innere des Druckraumes 21 eingreift und mit der Außenseite seiner Kappenstirnwand die Handhabe 6 bildet. Der Deckel 24 ist über eine axiale, selbsteinrastende Schnappverbindung 26 gegen Abziehen gegenüber dem Gehäuseteil 12 gesichert und trägt im Bereich seines offenen, das zugehörige Schnappglied aufweisenden Kappenrandes den gesonderten, napf- bzw. ringförmigen Kolben 25, der ebenfalls über eine Schnappverbindung 28 an dem Deckel 24 axial selbsteinrastend befestigt ist.

Am Kolbenboden des mit einer Ringlippe an der Kolbenlaufbahn 23 laufenden Kolbens 25 ist eine Rückstellfeder 27 mit einem Ende abgestützt, deren anderes Ende zentriert an der Querwand 22 unter Vorspannung abgestützt ist und die Kolbeneinheit zu der durch die Schnappverbindung 26 anschlagbegrenzten Ausgangsstellung belastet. Der Kolbenboden ist innerhalb der Schnappverbindung 28 von einem in die Druckkammer 21 mündenden Einlaßkanal durchsetzt, in welchem zweckmäßig etwa im Bereich der Kappenstirnwand ein Rückschlag- bzw. Einlaßventil 29 angeordnet ist, so daß durch eine Öffnung in der Kappenstirnwand bzw. der Handhabe 6 Luft von außen in den Druckraum 21 beim Rückhub angesaugt werden kann.

Die Magazinkammern 30 sind im wesentlichen in gleichen Winkelabständen in einem Kranz um die Zentralachse 10 angeordnet, durchgehend geradlinig ausgebildet, radial zur Zentralachse 10 ausgerichtet und gegeneinander dicht verschlossen, wobei die Länge jeder Magazinkammer 30 drei, vier oder sogar mehr als sechsfach größer als ihre lichte Weite sein kann, die zweckmäßig über ihre gesamte Länge glattwandig konstant ist. Jede Magazinkammer 30 ist aus zwei Rinnen- bzw. Schalenbegrenzungen zusammengesetzt, so daß eine einfache Herstellung der Bauteile 17, 18 als Spritzgußteile möglich ist.

Der Magazinkörper 16 ist mit einem an seinem inneren Ringumfang liegenden Radiallager 31 und einem an seinem äußeren Ringumfang liegenden Radiallager 32 so gelagert, daß durch diese Radiallager im wesentlichen alle Magazinkammern 30 gleichzeitig dicht verschlossen sein können. Ferner sind zur axialen Festlegung des Magazinkörpers 16 zwei Axiallager 33, 34 vorgesehen, von denen das Axiallager 33 durch ringförmig abgesetzten Dreheingriff des offenen Kappenrandes des Deckels 13 in das zugehörige Ende des Gehäuseteiles 12 gebildet ist, während das entgegengesetzt wirkende Axiallager 34 an einer dem Generator 4 bzw. der Querwand 22 zugekehrten Stirnseite des Magazinkörpers 16 angreift. Das Axiallager 33 bildet gleichzeitig ein Radiallager bzw. eine Zentrierung für den Deckel 13 gegenüber dem Gehäuseteil 12.

Der Magazinkörper 16 ist schrittweise um die Winkelteilung der Anordnung der Magazinkammern 30 mit einer Rastung 35 drehbar, die durch das Axiallager 34 gebildet sein kann, vollständig innerhalb des Gehäuseraumes 14 liegt und eine Drehbewegung nur in einer einzigen Drehrichtung nach Art eines Freilaufes zuläßt, der gegen die entgegengesetzte Drehrichtung formschlüssig sperrt. Der Magazinkörper 16 ist über eine ebenfalls innerhalb des Gehäuseraumes 14 liegende Kupplung 36 mit dem Deckel 13 bzw. der Handhabe 7 zumindest hinsichtlich der Schalt-Drehrichtung im wesentlichen starr verbunden. Diese Kupplung 36 ist zerstörungsfrei leicht trennbar und z.B. durch eine Bajonettkupplung zwischen der der Handhabe 5 zugekehrten Stirnseite des Magazinkörpers 16 und einer im Querschnitt mehreckigen Innenhülse des Deckels 13 gebildet, die frei von der Innenseit der Kappenstirnwand vorsteht.

Auf eine im Bereich des Innenumfangs von der Querwand 22 in den Gehäuseraum 14 vorstehende Muffe ist ein starr mit dem Grundkörper 8 verbundener, die Zentralachse 10 umgebender Innenkörper 37 mit einem Hülsenende aufgesteckt, der den Magazinkörper 16 durchsetzt und im Bereich seines anderen Hülsenendes ein Schnappglied einer Schnappverbindung 38 bildet, mit welcher der Deckel 13 gegen Abziehen vom Gehäuseteil 12 bzw. vom Axiallager 33 so gesichert ist, daß er nur bei Überschreiten einer vorbestimmten Abziehkraft axial vollständig abgenommen werden kann.

Die Schnappglieder der Schnappverbindung 38 greifen radial federnd drehbar ineinander und bilden daher eine Schnapplagerung für den Deckel 13 und den Magazinkörper 16. Nach Abziehen des Deckels 13 liegt der Magazinkörper 16 im Abstand vom offenen Deckelrand frei, so daß er durch eine Drehbewegung gegenüber dem Deckel 13 von diesem gelöst und gegen einen anderen Magazinkörper 16 ausgewechselt werden kann. Danach wird der Deckel 13 wieder auf den Grundkörper 8 aufgesetzt, wobei zuerst der Magazinkörper 16 in Eingriff mit seinen Radiallagern 31, 32 kommt, bis dann darauffolgend der Deckel 13 in das Lager 33 eingreift, so daß sich hier eine axiale Stecklagerung zum Auswechseln ergibt.

Der Innenkörper 37 bildet innerhalb seiner dem Radiallager 31 zugehörigen Außenhülse einen länglich napfförmig am Boden geschlossenen, hohlen Kernkörper 39, der durch die Muffe bzw. die zentrale Öffnung der Querwand 22 in den Druckraum 21 hineinragt und mit der zentralen Öffnung bzw. der Muffe eine ringförmige Auslaßöffnung für den Druckraum 21 begrenzt, die im Durchflußquerschnitt gegenüber einem darauffolgenden Abschnitt des Förderweges 20 verengt ist. Durch den einteilig bzw. dicht und etwa in der Ebene der Magazinkammern 30 an den Innenumfang der Außenhülse anschließenden Kernkörper 39 ist der Druckraum 21 druckdicht gegenüber dem Gehäuseraum 14 verschlossen.

Das Schaltwerk 40 zum Weiterschalten des Magazinkörpers 16 ist hier im wesentlichen ein unabhängig von sonstigen Betätigungen der Austragvorrichtung mit der Handhabe 7 zu betätigendes Schrittschaltwerk, dessen Schaltschritte durch die Rastung 35 bestimmt sind. Zu diesem Zweck weist der Magazinkörper 16 an einer Stirnseite sägezahnförmig in einem Kranz angeordnete Rastglieder 41 auf, entlang welchen mindestens ein federndes Rastglied 42 des Grundkörpers 8 läuft, das z.B. durch von der Querwand 22 abstehende Federzungen gebildet sein kann. In der jeweiligen Raststellung ist eine Magazinkammer 30 als Vorlagekammer so gegenüber dem Förderweg 20 ausgerichtet, daß sie als Kanalabschnitt an den Förderweg 20 angeschlossen ist und der gesamte, den Förderweg 20 durchströmende Medienstrom nur durch die Vorlagekammer 30 weitergeleitet werden kann. Die Magazinkammer 30 durchsetzt einen im wesentlichen zylindrischen Außenumfang 43 und einen im wesentlichen zylindrischen Innenumfang 44 des Magazinkörpers 16, wobei der jeweilige Umfang durch einen hülsenförmigen Lagerflansch gebildet sein kann, der über eine oder beide Stirnseiten des ringscheibenförmigen Abschnittes axial vorsteht.

Am Innenumfang 44 bildet die Magazinkammer 30 mit ihrem radial inneren Ende einen Einlaß 46 und am Außenumfang 43 mit ihrem radial äußeren Ende einen Auslaß 47, jeweils zur im wesentlichen dichten Verbindung mit einem anschließenden Abschnitt des Förderweges 20. Damit kein sich lockerndes Medium aus der Magazinkammer 30 zwischen die Lagerflächen des jeweiligen Lagers gelangen kann, kann der Einlaß 46 und/oder der Auslaß 47 jeweils mit einem Verschlußglied 45 verschlossen sein, das nicht oder unwesentlich über die zugehörige Durchtrittsfläche des Magazinkörpers 16 vorsteht und beispielsweise durch eine dünne, selbsthaftende oder durch Schweißung befestigte Folie gebildet sein kann, die zweckmäßig leicht reißt und/oder verhältnismäßig spröde ist. Das Verschlußglied 45 kann für alle Einlässe 46 bzw. Auslässe 47 gemeinsam in Form einer Banderole vorgesehen sein, die über den zugehörigen Umfang des Magazinkörpers 16 ununterbrochen durchgeht.

Zur Öffnung des jeweiligen Auslasses 47 bzw. Einlasses 46 kann jeweils eine Öffnungseinrichtung 50 bzw. 51 vorgesehen sein, deren Arbeitsbewegung zweckmäßig unmittelbar die Dreh-Schaltbewegung des Magazinkörpers 36 ist, so daß das am Grundkörper 8 gelagerte Öffnungswerkzeug 48 feststehend angeordnet sein kann. Dieses, zweckmäßig aus Stahl oder einem anderen sehr harten Werkstoff bestehende Öffnungswerkzeug 48 kann im Falle der Öffnungseinrichtung 51 durch einen Stabkörper gebildet sein, der in eine die Schnappverbindung 38 durchsetzende Längsnut im äußeren Mantel des Innenkörpers 37 eingesetzt ist. Das Öffnungswerkzeug kann aber auch, wie z.B. im Falle der Öffnungseinrichtung 50, ein sternförmiger Körper mit drei etwa radial zur Mittelachse der auf ihn ausgerichteten Magazinkammer 30 abstehenden Armen ausgebildet sein, der gegen den zugehörigen Umfang des Magazinkörpers 16 angelegt ist.

In jedem Fall weist das Öffnungswerkzeug 48 zweckmäßig eine Ritzschneide 52 auf, die ähnlich einem Scherwerkzeug in ein Gegenwerkzeug bzw. ein weiteres Öffnungswerkzeug 49 eingreift, das unmittelbar durch den Magazinkörper 16 bildet sein kann. Beispielsweise kann die Ritzschneide 52 in eine von dem Verschlußglied 45 überbrückte Umfangsnut des Magazinkörpers 16 eingreifen, so daß das Verschlußglied bei der Schaltbewegung zwangsläufig aufgeschlitzt und dadurch der Einlaß 46 bzw. der Auslaß 47 geöffnet wird.

Im Mantel der Außenhülse des Innenkörpers 37 ist eine an den ringförmigen Abschnitt des Förderweges 20 angeschlossene, radiale Durchgangs- bzw. Anschlußöffnung 53 vorgesehen, die im wesentlichen achsgleich zu der in Austragstellung befindlichen Magazinkammer 30 ausgerichtet ist und sich dann nach Art eines Schieberventiles mit dem Einlaß 46 wenigstens teilweise deckt. Auf einem größeren Teil ihres Umfangs ist die Anschlußöffnung 53 durch das zugehörige Öffnungswerkzeug begrenzt, wobei ihre Durchflußweite kleiner als die des Einlasses 46 bzw. der Magazinkammer 30 sein kann. Im Bereich des Auslasses 47 ist die zugehörige Anschlußöffnung 54 zwischen den Sternarmen des Öffnungswerkzeuges begrenzt, die die Ritzschneide 52 im Knotenpunkt bzw. Zentrum des Sternkörpers aufweisen. Die Sternarme können im Querschnitt kantig, z.B. mit einer entgegen Strömungsrichtung weisenden Profilkante ausgebildet sein, so daß sich Prall- bzw. Leit- und Lockerungsflächen ergeben, durch welche das entlangströmende Medium aufgewirbelt und ggf. entklumpt wird.

Die Anschlußöffnung 54 ist durch das innere Ende eines geradlinigen, zur Vorlagekammer 30 annähernd achsgleichen Endkanales 55 gebildet, der an den Auslaß 47 bzw. die Anschlußöffnung 54 mit demgegenüber größerem Durchflußquerschnitt anschließt und dann in einen konisch kontinuierlich verengten Kanalabschnitt 56 übergeht, der mit seinem engsten Bereich die zur Vorlagekammer 30 koaxiale Austragöffnung 11 bildet.

Der Endkanal 55 ist von einer zur Zentralachse 10 quer liegenden Kupplung 57 umgeben, die an der Außenseite des Gehäuses 9 liegt bzw. über dessen Außenmantel 58 etwa radial vorsteht und ein hülsenförmig mit dem Grundkörper 8 verbundenes Kupplungsglied aufweist. Auf dieses Kupplungsglied ist von außen ein den Endkanal 55 bzw. die Austragöffnung 11 bildender, langgestreckter Austragstutzen 60 aufsetzbar und mit einer Steck- bzw. Schnappverbindung 59 zu sichern, die zweckmäßig durch die Kupplung 57 gebildet ist. Der Austragstutzen 60 weist außer einem das Kupplungsglied außen mit der Schnappverbindung 89 übergreifenden Mantel einen in das Kupplungsglied eingreifenden Innenmantel auf, dessen Ende das Öffnungswerkzeug der Öffnungseinrichtung 50 gegen den Umfang des Magazinkörpers 16 anlegt.

Zum Betrieb der Austragvorrichtung wird in der beschriebenen Weise ein Magazinkörper 16 mit gefüllten Magazinkammern 30 eingesetzt und dann die jeweils gewünschte Magazinkammer 30 ggf. anhand einer von außen erkennbaren Anzeige durch manuelles Drehen der Handhabe 7 gegenüber dem Gehäuseteil 12 auf den Leitungsanschluß an den Förderweg 20 bzw. die Anschlußöffnungen 53, 54 ausgerichtet. Danach wird die Austragöffnung 11 in den Bereich der Anwendungszone gebracht, und es wird der Kolben 25 gegen die Querwand 22 manuell verschoben. Dadurch strömt aus dem Druckraum 21 komprimierte Luft zur Anschlußöffnung 53 und drückt gegen den Einlaß 46.

Sobald ein bestimmter Überdruck erreicht ist, wird die pfropfartige Füllung der Magazinkammer 30 in Austragrichtung ausgeschoben, wobei sie an der Ritzschneide 52 und den Sternarmen aufgelockert und dann vom nachfolgenden Gasstrom unter Verwirbelung im Endkanal 55 durch die Austragöffnung 11 ausgestoßen wird. Nach Freigabe der Handhaben 5, 6 kehrt die Austragvorrichtung 1 unter der Kraft der Rückstellfeder 27 wieder in ihre Ausgangslage zurück, und es kann die nächste Magazinkammer 30 zur Bildung einer Vorlagekammer durch Drehen in Austragstellung bewegt und dann ebenfalls entleert werden. Die Austragvorrichtung 1 arbeitet in jeder Lage gleich gut, auch in Kopflage. Die Gesamtlänge der langestreckten Vorrichtung kann unter 10 cm. liegen.

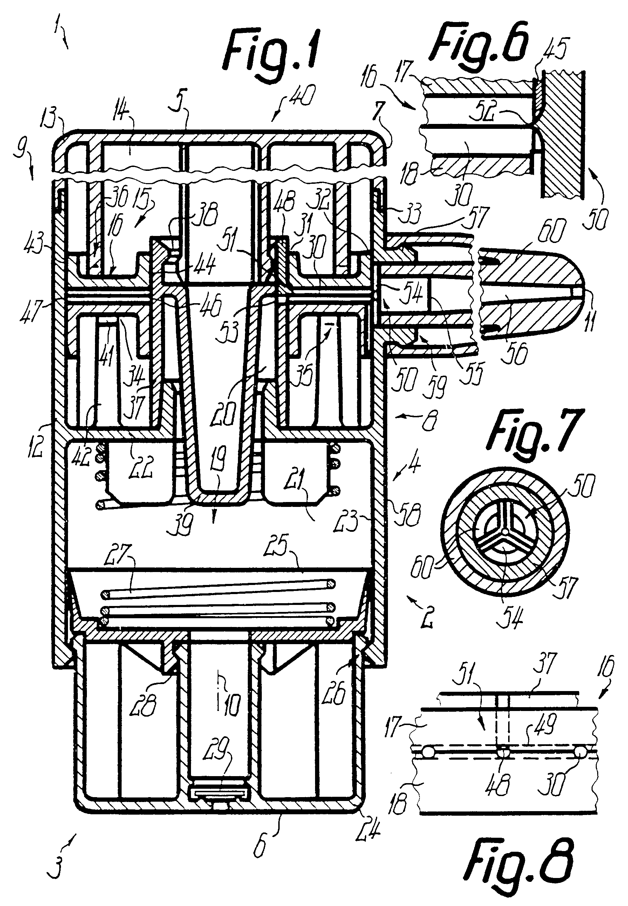

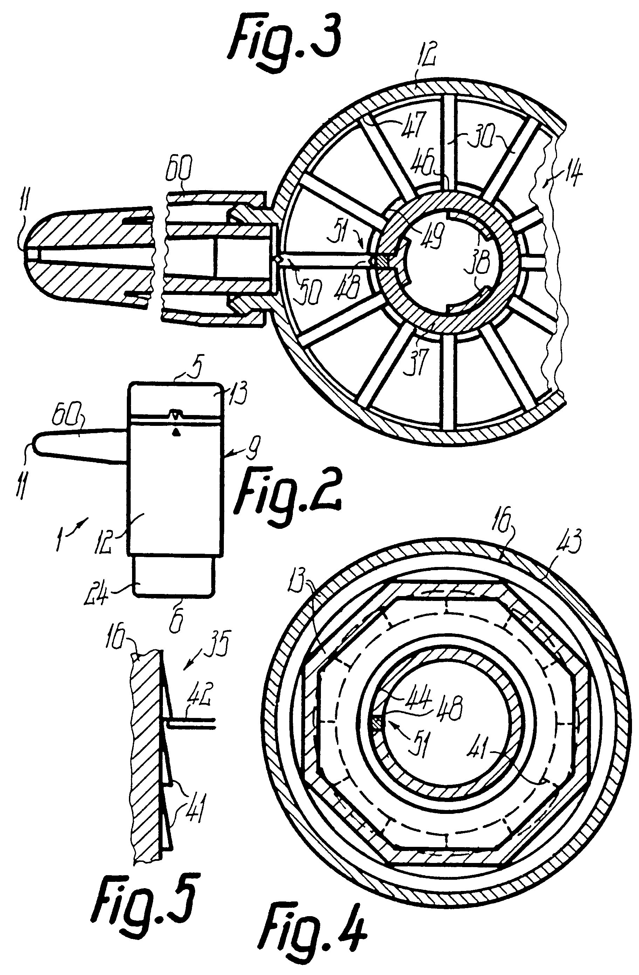

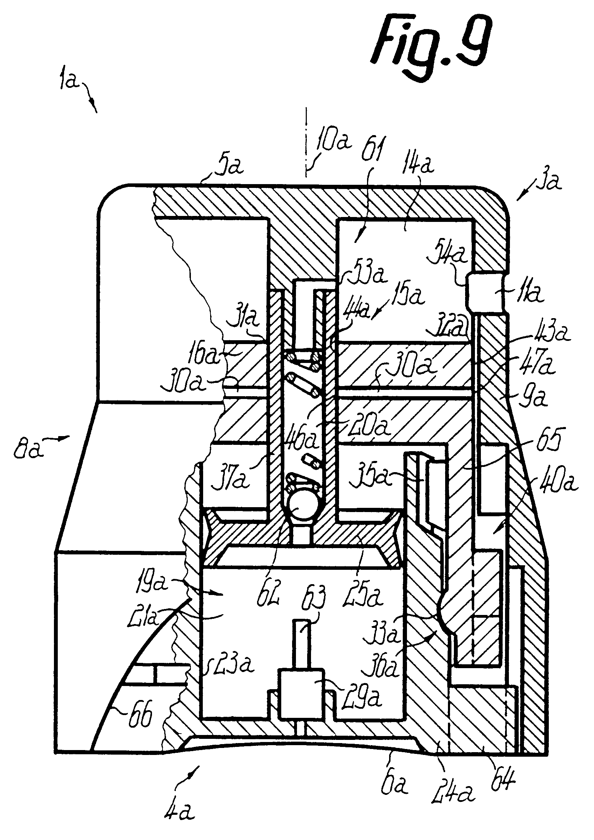

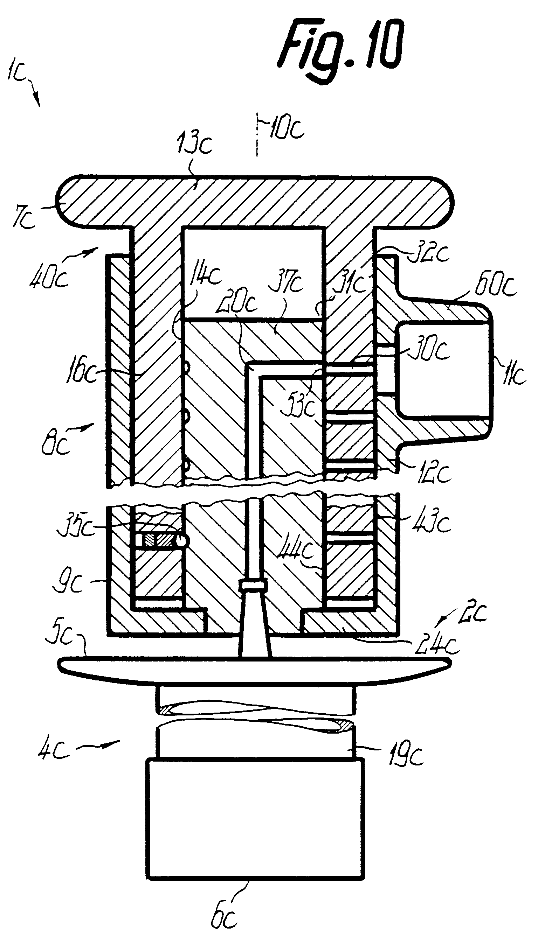

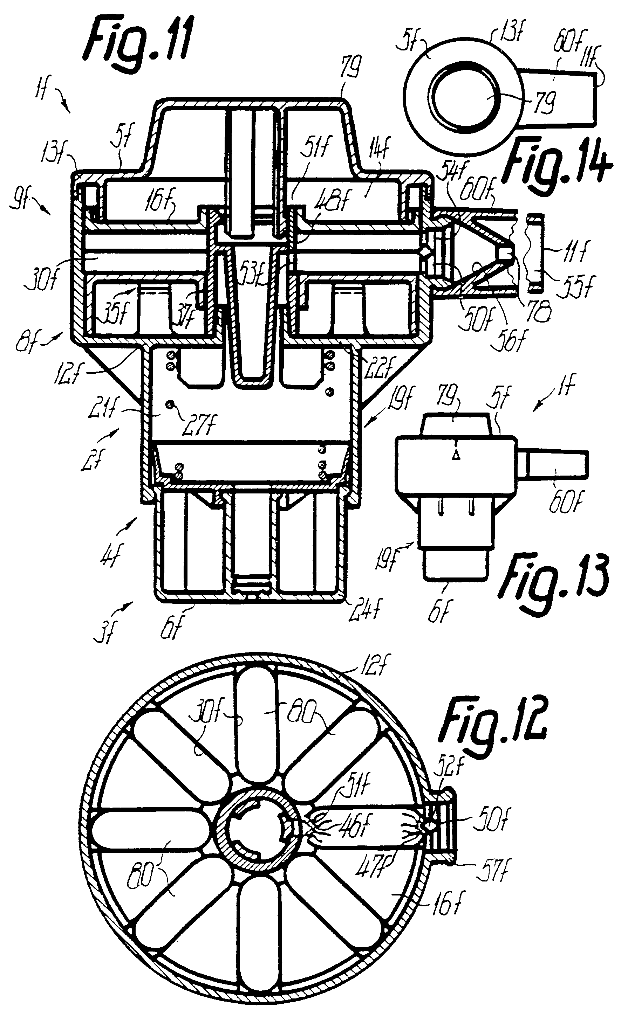

In den Figuren 9 bis 26 sind für einander entsprechende Teile die gleichen Bezugszeichen wie in Fig. 1, jedoch mit unterschiedlichen Buchstabenindizes verwendet, wobei alle Beschreibungsteile sinngemäß für alle Ausführungsformen gelten.

Gemäß Fig. 9 nimmt der gehäuse- bzw. kappenförmige Grundkörper 8a im wesentlichen alle Bauteile der Austragvorrichtung 1a in seinem Innern auf, so daß sich eine sehr glatte und kontinuierliche Außenfläche ergibt. Von der Kappenöffnung des Grundkörpers 8a her greift ein entgegengesetzt ebenfalls etwa kappenförmiger Deckel- bzw. Bauteil 24a ein, der mit einem im Bereich eines Endes vorgesehenen, erweiterten Außenbund 64 durch Nut- und Federführung gegenüber dem Grundkörper 8a verdrehgesichert, jedoch zur Durchführung der Austragbetätigung axial verschiebbar ist. Über die innere Stirnfläche des Außenbundes 64 steht ein Mantelansatz vor, welcher die Kolbenlaufbahn 23a bildet, so daß hier der Druckraum 21a von dem Deckel 24a gebildet und gegenüber dem Grundkörper 8a verschiebbar ist.

Zwischen den Außenumfang des Mantelansatzes und den Innenumfang des Grundkörpers 8a greift der Magazinkörper 16a mit einer über seinen ringscheibenförmigen Magazinbereich axial vorstehenden Steuerhülse 65 ein, mit welcher der Magazinkörper 16a am Außenumfang des Mantelansatzes drehbar gelagert ist. Das zugehörige Axiallager 33a ist durch einen in eine Ringnut eingreifenden Ringwulst gebildet, so daß es gleichzeitig ein Radiallager sowie in Form einer axial zu trennenden federnden Schnappverbindung die Kupplung 36a zur Lösung des Magazinkörpers 16a von dem Deckel 24a bildet. Benachbart zum Lager 33a ist im Bereich des freien Endes des Mantelansatzes die Freilaufrastung 35a zwischen dem Außenumfang des Mantelansatzes und dem Innenumfang der Steuerhülse 65 vorgesehen, durch welche die Schaltschritte des Schaltwerkes 40a wenigstens teilweise definiert sind.

Ein Schalttrieb des Schaltwerkes 40a ist zwischen dem Außenumfang der Steuerhülse 65 und dem Innenumfang des Grundkörpers 8a vorgesehen und z.B. durch über den Umfang verteilte Schrägflächen an beiden Bauteilen gebildet, durch welche beim gegenseitigen Axialhub der Magazinkörper 16a entgegen der Kraft der Rastung 35a um einen Schaltschritt weiterbewegt wird. Die Verdrehsicherung zwischen dem Grundkörper 8a und dem Deckel 24a liegt am Innenumfang des Grundkörpers 8a und kann bis in den Bereich eines Außenbundes der Steuerhülse 65 reichen, welcher die zugehörigen Schrägflächen aufweist.

Der Kolben 25a ist in diesem Fall an einer frei von der Innenseite der Kappenstirnwand des Grundkörpers 8a abstehenden Kolbenstange 37a angeordnet, welche den Magazinkörper 16a außer drehbar auch axial verschiebbar durchsetzt, da der Magazinkörper 16a axial gegenüber dem Deckel 24a durch das Lager 33a festgelegt ist. Der Kolben 25a sowie die Kolbenstange 37a sind von dem Förderweg 20a durchsetzt, der in die am Außenumfang der Kolbenstange 37a liegende Anschlußöffnung 53a übergeht. Im Förderweg 20a ist ein Ventil 62 als Auslaßventil für den Druckraum 21a vorgesehen, das einen zur Schließstellung federbelasteten Ventilkörper 62 in der Kolbenstange 37a bzw. in der Kolbeneinheit aufweist.

Dem Ventilkörper ist ein Ventilöffner 63 zugeordnet, welcher kurz vor oder am Ende des Arbeitshubes das Ventil 62 mechanisch dadurch öffnet, daß der Ventilkörper an einem von der gegenüberliegenden Stirnwand des Druckraumes 21a abstehenden Öffnerdorn anschlägt. Dieser Ventilöffner 63 kann eine Baueinheit mit dem Einlaßventil 29a bilden, das seinerseits einschließlich eines Ventilgehäuses in eine Halterung der Stirnwand eingesetzt sein kann.

In Ausgangslage gemäß Fig. 9 liegt die jeweils als nächste zu leerende Magazinkammer 30a bzw. deren Einlaß 46a und/oder Auslaß 47a axial versetzt gegenüber der zugehörigen Anschlußöffnung 53a bzw. 54a, und zwar etwa um den Arbeitshub der Luftpumpe 19a; ferner liegt sie in Drehrichtung um einen Schaltschritt vorversetzt. Wird nun der Bauteil 24a in den Grundkörper 8a entgegen der Kraft der nicht näher dargestellten Rückstellfeder hineingedrückt, so nimmt er den Magazinkörper 16a axial mit. Gleichzeitig kommen die Schrägflächen des Schaltwerkes 40 in Eingriff miteinander, so daß die bei diesem Betätigungszyklus zu entleerende Magazinkammer 30a unter Überwindung der Rastung 35a um einen Schaltschritt weitergedreht wird, bevor der Einlaß 46a die Anschlußöffnung 53a erreicht hat.

Im weiteren Verlauf der Axialbewegung läuft der Innenumfang 44a über die Anschlußöffnung 53a, so daß diese nun nach Art einer Schiebersteuerung im wesentlichen dicht geschlossen ist und erst freigegeben wird, wenn sie in Deckung mit dem Einlaß 46a gelangt. In diesem Augenblick oder kurz zuvor wird das Ventil 62 entgegen der Kraft seiner Ventilfeder geöffnet, so daß das hochkomprimierte Gas aus dem Druckraum 21a durch den Förderweg 20a schlagartig in die entsprechende Magazinkammer 30a entweicht. In ähnlicher Weise kann durch Schiebersteuerung auch die Anschlußöffnung 54a zunächst durch den Außenumfang 43a des Magazinkörpers 16a geschlossen und dann in Deckung mit dem Auslaß 47a gebracht werden. Hier sind die Anschlußöffnung 54a und die Austragöffnung 11a durch das innere bzw. äußere Ende einer Durchgangsöffnung im Mantel des Grundkörpers 8a gebildet. Damit die Handhabe 6a über den gesamten Arbeitshub gut zugänglich ist, ist im Mantel des Grundkörpers 8a mindestens ein Finger-Ausschnitt 66 vorgesehen, der von der offenen Stirnfläche des Grundkörpers 8a ausgeht und dessen Höhe etwa dem Arbeitshub entspricht.

Die Austragvorrichtung kann auch in nicht dargestellter Weise etwa achsparallel zur Zentralachse angeordnete Magazinkammern aufweisen, die die beiden Stirnflächen des Magazinkörpers durchsetzen bzw. durch die Lagerflächen der an diesen Stirnseiten liegenden Axiallager verschlossen sind. Auch die Anschlußöffnungen liegen in den entsprechenden Stirnflächen des z.B. scheibenförmigen Grundkörpers und des ebenfalls im wesentlichen scheibenförmigen gegenüberliegenden Körpers , der in diesem Fall einteilig mit dem Austragstutzen ausgebildet sein kann.

Zwischen dem Grundkörper und dem gegenüberliegenden Körper, die gegeneinander durch einen ein Radiallager bildenden Bolzen gespannt und verdrehgesichert sind, liegt der auf dem Bolzen drehbar gelagerte Magazinkörper, dessen Außenumfang wenigstens auf einem Teil seiner Erstreckung zugänglich ist, um als Handhabe zum Drehen zu dienen. Der Generator bzw. die Luftpumpe ist in diesem Fall durch eine Leitungs-Steckkupplung ggf. leicht und zerstörungsfrei, lösbar mit der Austragvorrichtung verbunden, so daß diese nach Entleerung gegen eine neue Austragvorrichtung ausgetauscht werden kann, welche dann wieder durch Steckverbindung mit der Luftpumpe , einer Treibgasdose oder dgl. zu verbinden ist.

Der Generator liegt dabei etwa um den Radius des Kranzes von Magazinkammern exzentrisch zur Dreh- bzw. Zentralachse . Entsprechend exzentrisch ist auch die Rastung zwischen aneinander laufenden Stirnflächen des Magazinkörpers und des Grundkörpers vorgesehen, wobei der Grundkörper in einem Kranz radial außerhalb der Magazinkammern Rastvertiefungen aufweist, in die eine federbelastete Rastkugel eingreift.

Der Einlaß bzw. der Füllraum der jeweiligen Magazinkammer kann gegenüber der zugehörigen Stirnfläche zurückversetzt sein, so daß ein zu ihm etwa achsgleicher, jedoch erweiterter Gehäuseraum für die Aufnahme eines Siebes und/oder einer Dichtung gebildet ist. Das Sieb liegt in Form einer flachen Siebscheibe an einer den Einlaß umgebenden Ringschulter des Gehäuseraumes an, der durch eine entsprechende Vertiefung in der zugehörigen Durchsetzungs- bzw. Stirnfläche des Magazinkörpers gebildet ist.

Die als O-Ring ausgebildete, gummielastische Dichtung drückt das Sieb gegen die Ringschulter und läuft mit ihrer davon abgekehrten Seite an der zugehörigen Stirnfläche des Grundkörpers mit axialer Pressung, so daß sie bei Ausrichtung gegenüber der Anschlußöffnung eine Abdichtung des Förderweges bildet. Eine entsprechende Dichtung bzw. ein Sieb könnte auch im Bereich des Auslasses oder der Anschlußöffnung vorgesehen sein. Durch das Sieb ist ein Verschluß des zugehörigen Endes der Magazinkammer gebildet, welcher zwar für das Fördergas, nicht jedoch für das Medium ohne weiteres durchlässig ist. Der Grundkörper und der Bauteil können auch einteilig miteinander ausgebildet sein bzw. den Magazinkörper auf einem Teil seines Außenumfanges übergreifen und/oder gehäuseartig abdecken.

Gemäß Fig. 10 ist der Magazinkörper 16c zur Weiterschaltung linear und etwa parallel zur Zentralachse 10c verschiebbar geführt, wobei die Magazinkammern 30c in dieser Längsrichtung und parallel zueinander hintereinander vorgesehen sind. Der Magazinkörper 16c ist hülsenförmig ausgebildet und erstreckt sich annähernd über die gesamte Länge des Grundkörpers 8c, des Gehäuses 9c bzw. des im Querschnitt ringförmigen Gehäuseraumes 14c, der am Außenumfang von dem hülsen- bzw. napfförmigen Gehäuseteil 12c und am Innenumfang von dem etwas kürzeren Innenkörper 37c begrenzt ist, welcher auch einteilig von der Stirn- bzw. Bodenwand 24c des Gehäuseteiles 12c abstehen kann und von dem Förderweg 20c durchsetzt ist. Der Magazinkörper 16c ist von der offenen Napfseite her in den Gehäuseteil 12c eingesetzt und weist an seinem außerhalb des Gehäuseteiles 12c liegenden Ende einen über seinen Außenumfang vorstehenden Tellerbund 13c auf, welcher die Handhabe 7c zum Axialverschieben gegenüber dem Grundkörper 8c bildet.

An der von der Anschlußöffnung 53c abgekehrten Seite ist die Rastung 35c für den Magazinkörper 16c vorgesehen, die z.B. ein im Mantel des Magazinkörpers 16c etwa radial federnd belastetes Rastglied sowie für jede Magazinkammer 30c eine Rastöffnung am Außenumfang des Innenkörpers 37c aufweist. Ist der Magazinkörper 16c gegenüber dem Grundkörper 8c nicht verdrehgesichert, sondern drehbar gelagert, so können in jeder Ebene quer zur Zentralachse 10c ähnlich Fig. 1 mehrere Magazinkammern 30c bzw. Rastungen 35c vorgesehen sein, so daß sowohl durch Drehen als auch durch axiales Verschieben weitergeschaltet werden kann und ggf. in einzelnen Ebenen unterschiedliche Medien oder Dosen zum Austrag bereitgehalten werden können. Der Austragstutzen 60c ist einteilig mit dem Grundkörper 8c bzw. dem Innenkörper 37c ausgebildet, die auswechselbar mit dem Generator 4c zu verbinden sind.

Die Magazinkammern können auch mit ein Nachfülleinrichtung aus einem Speichermagazin selbsttätig befüllt werden, das eine gegenüber der Aufnahmekapazität der Magazinkammer bzw. -kammern wesentlich größere Speicherkapazität hat. Mindestens ein schachtförmiges, über seine Länge annähernd konstante Innenquerschnitte aufweisendes Speichermagazin steht über die vom Magazinkörper abgekehrte Stirnseite des Grundkörpers vor, so daß es z.B. in etwa gleicher Richtung wie der Austragstutzen oder der Generator frei ausragen kann und von der zugehörigen Stirnseite her an den Magazinkörper anzuschließen ist.

Der gegenüber der jeweiligen Magazinkammer etwa gleich weite Speicherraum des Speichermagazines schließt über eine kurze Übertrittsöffnung an die jeweils in Deckung mit ihm stehende Magazinkammer an, wobei diese Öffnung im Querschnitt gegenüber dem Speicherraum und/oder der Magazinkammer geringfügig verengt sein kann, so daß das Medium beim Übertritt geringfügig verdichtet wird und sich dann in der Magazinkammer wieder lockern bzw. aufweiten kann. An dem von der Übertrittsöffnung abgekehrten Ende ist der Speicherraum mit einem verschiebbar geführten Kolben begrenzt, der mit der Abnahme der Speichermenge durch Gewichtskraft, Federkraft und/oder Rüttelbewegungen nachläuft, so daß das gespeicherte Medium stets in Richtung zur Übertrittsöffnung belastet und in gleichmäßig verdichteter Struktur im Speicherraum gehalten wird.

Die zum Speicherraum koaxiale Übertrittsöffnung kann z.B. um etwa 180° oder einen davon abweichenden Bogenwinkel um die Drehachse gegenüber den Anschlußöffnungen versetzt und so angeordnet sein, daß bei Ausrichtung einer Magazinkammer gegenüber den Anschlußöffnungen eine andere Magazinkammer an eine oder mehrere Übertrittsöffnungen angeschlossen ist. Der Magazinkörper kann nur eine einzige, nur zwei oder mehr Magazinkammern aufweisen. Die den Magazinkörper an beiden Stirnseiten übergreifenden bzw. lagernden Gehäuseteile können einteilig miteinander ausgebildet bzw. durch den Grundkörper gebildet sein, der auch ein den Magazinkörper auf dem größten Teil seines Umfanges umgebendes Gehäuse sein kann. Vor dem ersten Gebrauch können alle Magazinkammern sowie das Speichermagazin voll gefüllt sein, so daß insgesamt eine verhältnismäßig große Speicherkapazität gegeben ist. Auch das Speichermagazin selbst kann auswechselbar am Grundkörper gehaltert und/oder nachfüllbar ausgebildet sein, wofür z.B. an einer von der Übertrittsöffnung abgekehrten Ende mit einem leicht lösbaren Deckel versehen sein kann.

Ferner kann die Vorlagekammer durch einen dem Ventil bzw. der zugehörigen Ventilfeder unmittelbar nachgeschalteten Abschnitt des Förderweges gebildet sein, der von der Vorlage zur Austragöffnung geradlinig und auf dem größten Teil seiner Länge mit konstanter Weite durchgeht. In der Wandung des rohrförmigen Innenkörpers ist eine geradlinige, annähernd radiale Übertrittsöffnung vorgesehen, die den Innenumfang sowohl der Vorlagekammer als auch des etwa gleich weiten Speichermagazines durchsetzt, welches von der Vorlagekammer nur durch eine einzige Zwischenwand getrennt ist. Zur Überführung einer Mediendosis von dem Speichermagazin durch die Übertrittsöffnung in die Vorlagekammer ist ein stab- bzw. bolzenförmiger Schieber vorgesehen, dessen freies Förderende in Ausgangsstellung nahe dem Boden innerhalb des Mediums in dem Speichermagazin liegt. Dieses Ende des Schiebers kann einen mit seiner Tragfläche etwa in Ausströmrichtung aus der Vorlagekammer weisenden, ausgerundeten Schöpfer, einen Löffel oder eine Zuspitzung aufweisen, so daß es das vom Speichermagazin übernommene Medium keilartig in die Übertrittsöffnung einschieben und dann nach Art eines Vorlagebodens in der Endlage innerhalb der Vorlagekammer tragen kann. Der Löffel ist dabei so klein, daß er über den größten Teil des Umfanges der Vorlagekammer im Abstand von deren Innenumfang liegt und umströmt werden kann.

Der Schieber ist etwa radial zur Zentralachse in einer Führung des Grundkörpers gegen die Kraft einer Rückstellfeder verschiebbar gelagert und ragt mit einem Ende bzw. Betätigungsabschnitt über den Außenmantel des Grundkörpers vor. Die Endfläche dieses Betätigungsabschnittes liegt an einer Steuerkurve an, welche durch den Innenumfang des Mantels der zweiten Handhabe gebildet ist, welcher auch die Kolbenlaufbahn bildet. Je nachdem, ob die Steuerkurve als Umfangs- und/oder als Axialkurve ausgebildet ist, führen Dreh- und/oder Axialbewegungen dieses Bauteiles der zweiten Handhabe gegenüber dem Grundkörper zu quer dazu liegenden Linearbewegungen des Schiebers.

Beim Betätigungshub des Generators wird zunächst der Löffel durch die eng an seinen Querschnitt angepaßte Übertrittsöffnung mit Medium in das Zentrum der Vorlagekammer überführt und gleichzeitig im Druckraum die Luft vorkomprimiert, wonach der Löffel in dieser Austraglage stehen bleibt und dann das Ventil durch den Ventilöffner geöffnet wird, so daß die Luft mit hoher Strömungsgeschwindigkeit den Löffel von der Rückseite her umströmt und das Medium mitreißt. In der Austragstellung bildet der Löffel somit als Verdrängungskörper eine Verengung des Durchflußquerschnittes des Förderweges nur im Bereich der Vorlagekammer.

Der Pumpkolben ist über die Schnappverbindung an dem in Strömungsrichtung des Förderweges hinteren Ende des Grundkörpers unmittelbar benachbart zur Querwand befestigt, die nur etwa um die Länge der Schnappverbindung von diesem Ende entfernt liegt. Der Pumpkolben kann dabei etwa gleiche Außenweite wie der Grundkörper aufweisen, welcher am Außenmantel von der Kolbenlaufbahn und der Steuerkurve der Schalteinrichtung für den Schieber übergriffen wird. Der Generator bzw. der Druckraum liegt dadurch unmittelbar benachbart zu diesem Ende des Grundkörpers bzw. des Gehäuseraumes.

Das Medium im Speichermagazin wird zwar durch einen Kolben zum Löffel hin belastet, jedoch ist es zweckmäßig, zur hohlraumfreien Bereitstellung des Mediums im Bereich des Löffels eine Rüttelvorrichtung vorzusehen, welche aufgrund einer Ratschenzahnung oder dgl. selbsttätig bei der Hubbewegung und/oder durch gesonderte Betätigung Vibrationen erzeugt, die auf das Speichermagazin bzw. den zugehörigen Bauteil wirken. Statt einer Ratschenzahnung zwischen den gegeneinander bewegbaren Hub-Bauteilen kann auch unmittelbar an einem dieser Bauteile, z.B. am Grundkörper , eine Rüttelratsche mit bei Betätigung einander überspringenden Zähnen einer Zahnung vorgesehen sein. Auf einem gezahnten, unmittelbar an den Deckel anschließenden Abschnitt des Außenumfanges des Grundkörpers ist ein mit einer Innenzahnung versehener Ring drehbar gelagert, der durch Greifen am Außenumfang manuell gedreht werden kann, so daß die genannten Vibrationen erzeugt werden, welche das Medium im Speichermagazin um den Löffel herum ausreichend verdichten. Das Speichermagazin liegt achsparallel zum in Querrichtung benachbarten Abschnitt des Förderweges innerhalb des Gehäuseraumes.

Die Austragvorrichtung 1f nach Fig. 11 ist ähnlich derjenigen nach den Figuren 1 bis 8 ausgebildet, jedoch dafür vorgesehen, in den Magazinkammern 30f das Medium nicht unmittelbar, sondern vorverpackt in dicht geschlossenen, langgestreckten Kapseln 80 aufzunehmen, die über ihre Länge etwa konstante, zylindrische Außenweite sowie ballig abgerundete Enden haben und zweckmäßig aus einem feuchtigkeitsstabilen, brüchigen Kunststoff bestehen. Das Öffnungswerkzeug der jeweiligen Öffnungseinrichtung 50f bzw. 51f weist einen in den bogenförmigen Schaltweg dieses Endes ragenden Spitzdorn 52f auf, der zweckmäßig im Querschnitt mehreckig, z.B. drei- oder viereckig, ist. Beim Einrücken des Kapselendes in die Austragstellung läuft es auf den Spitzdorn 52f auf, so daß es durch splitternde oder nicht splitternde und nur unter Biegeverformung sowie Rißbildung erfolgender Beschädigung geöffnet wird und dadurch den Einlaß 46f bzw. den Auslaß 47f bildet. Nach dem Austrag verbleiben die leeren Kapeseln in dem Magazinkörper 16f, der in der beschriebenen Weise gegen einen gefüllten ausgewechselt werden kann.

Die Anschlußöffnung 54f mündet in diesem Fall in eine wesentlich verengte Düse 78, die achsgleich zur Anschlußöffnung 54f liegt, an die spitzwinklig konische Verengung 56f anschließt und einen Axialabstand von der Anschlußöffnung 54f aufweist, der etwa in der Größenordnung von deren Weite liegt; der Axialabstand zur Austragöffnung 11f ist demgegenüber wesentlich größer. Die Düse 78 mündet etwa zentrisch und achsgleich in den gegenüber ihr bzw. gegenüber der Anschlußöffnung 54f wesentlich weiteren, bis einschließlich zur Austragöffnung 11f nur unwesentlich oder nicht verengten Endkanal 55f, in den sie den zunächst verdichteten Gemischstrom nach Art einer Zerstäuberdüse abgibt. Der Endkanal 55f bzw. die Austragöffnung 11f und der Austragstutzen 60f können Flachquerschnitte aufweisen, so daß sich ein gut zwischen die Lippen einzuführendes Mundstück ergibt.

Der Generator 4f weist eine gegenüber dem Grundkörper 8f und dem Gehäuseraum 14f reduzierte Außenweite auf, die bis zur zugehörigen Seite der Querwand 22f reicht, so daß der Gehäuseteil 12f zwei in der Weite gegeneinander abgesetzte Abschnitte etwa gleicher Länge bildet. Im Zentrum der Stirnwand des Deckels 13f steht ein napfförmiger Vorsprung 79 axial vor, so daß die Handhabe 5f anschließend an den Außenumfang des Gehäuses 9f ringförmig ausgebildet ist. Dadurch läßt sich die Austragvorrichtung 1f besonders gut mit einer Hand greifen, ausgerichtet führen und betätigen.

Die erste Handhabe kann auch näher bei dem der zweiten Handhabe zugekehrten Ende des Grundkörpers bzw. des Gehäuses als beim anderen Ende liegen, wobei sie an das hintere Ende des Grundkörpers körpers anschließen kann. Die erste Handhabe steht nach Art eines Quersteges oder einer Ringscheibe zumindest an zwei voneinander abgekehrten Seiten über den Außenumfang des Gehäuses vor, so daß an ihr Zeige- und Mittelfinger unter Zwischenlage des Gehäuses abgestützt werden können, während der Daumen an der zweiten Handhabe abzustützen ist. Der Grundkörper ist annähernd stabförmig langgestreckt in der Zentralachse ausgebildet und weist nahe benachbart zu seinem der Austragöffnung zugehörigen Ende in seinem Mantel als Durchbruch ein Fenster auf, in dessen Bereich der kreisscheibenförmige Magazinkörper exzentrisch und etwa parallel zur Zentralachse drehbar gelagert sein kann.

Die durch einen Lagerzapfen gebildete Drehachse liegt zwischen dem Innen- und dem Außenumfang des Gehäusemantels, so daß mindestens eine nicht in Austragstellung stehende Magazinkammer teilweise oder ganz an der Außenseite des Gehäuses bzw. innerhalb des Fensters liegen kann und der Magazinkörper zur Weiterschaltung unmittelbar an dieser Außenseite zugänglich ist. Die Magazinkammer bzw. der Magazinkörper ist kürzer als die jeweilige Kapsel , so daß diese mit einem oder beiden Enden über den Magazinkörper etwa gleich weit mindestens mit ihrem balligen Ende oder weiter vorsteht und dabei durch Umfangsklemmung gegenüber der Magazinkammer lagegesichert ist.

In der Austragstellung liegt das einlaßseitige Ende der jeweiligen Kapsel in einer schalenförmig die Anschlußöffnung umgebenden Vertiefung, welche die Kapsel bzw. den Magazinkörper nach Art einer federnd überwindbaren Rastung lagesichert, wobei hier zur Rastung die federnden Verformungseigenschaften der Kapsel herangezogen sind. Das auslaßseitige Ende der Kapsel kann in der Austragstellung innerhalb des Endkanales mit einem Abstand von der Austragöffnung vorgesehen sein, der in der Größenordnung der Länge der Kapsel liegt oder kleiner ist. Die Anschlußöffnung ist wesentlich enger als der die Kapsel ringförmig mit Radialabstand umgebende Abschnitt des Endkanales. Das Fenster kann so eng ausgebildet sein, daß es durch den Magazinkörper bzw. mindestens eine Kapsel in jeder Austragstellung im wesentlichen geschlossen bzw. gegenüber dem Kanal abgedichtet ist.

Zur Öffnung des Einlasses und des Auslasses der jeweiligen Kapsel ist eine Öffnungseinrichtung vorgesehen, die als in sich geschlossene Baugruppe zum Öffnen an den Grundkörper angesetzt und nach dem Öffnen wieder vollständig entfernt oder zur Seite geschwenkt werden kann, falls sie mit ihrem Basiskörper zwischen einer Öffnungsstellung und einer Ruhestellung bewegbar, z.B. schwenkbar am Grundkörper, gelagert ist. Der Basiskörper kann als annähernd hülsenförmiger Steckkörper ausgebildet sein, der mit einem reduzierten Steckvorsprung in die Austragöffnung bzw. den Endkanal etwa parallel zur Zentralachse zentriert eingesteckt werden kann. Im Steckkörper ist als Öffnungswerkzeug eine Stechnadel gegen die Kraft einer Rückstellfeder mit einer Handhabe verschiebbar gelagert, die dann etwa achsgleich zur in Austragstellung stehenden Kapsel liegt.

Die Stechnadel kann so weit durch die gesamte Kapsel bewegt werden, daß sie zuerst das auslaßseitige Ende und dann das einlaßseitige Ende durchsticht bzw. öffnet, wobei ein vom dadurch verdichteten Medium über den Umfang begrenzter Längskanal geschaffen wird, welcher den Einlaß mit dem Auslaß verbindet. Die Stechnadel weist zweckmäßig eine spitzwinklig konische Endspitze auf und ist nur so weit axial bewegbar, daß sie das einlaßseitige Ende der Kapsel lediglich mit einem Teil der Länge der Spitze durchdringt. Dadurch wird der Einlaß auf geringerer Weite als der Auslaß aufgrund einer entsprechenden Anschlagbegrenzung der Stechnadel geöffnet.

Der Grundkörper kann hier von der End- bzw. Austragöffnung einteilig bis zum davon abgekehrten Ende des Druckraumes durchgehen, der am zugehörigen Ende des Grundkörpers zur Anschlagsicherung des Pumpkolbens in der Ausgangstellung mit einem ringförmigen Deckel verschlossen ist. Dieser wird von der Kolbenstange durchsetzt, welche einteilig mit dem Pumpkolben und/oder der zweiten Handhabe ausgebildet sein kann. Der zwischen dem Druckraum und der Anschlußöffnung liegende Abschnitt des Förderweges ist geradlinig und einschließlich der Anschlußöffnung von konstanter Weite. Die Anschlußöffnung bzw. die diese umgebende Vertiefung kann durch das Ende eines frei vorstehenden Hülsenansatzes gebildet sein, welcher im Radialabstand ringförmig von einem auch gegenüber der Kapsel weiteren Innenumfang umgeben ist, dessen in Austragrichtung vorderes Ende jedoch zweckmäßig durch die zugehörige Stirnfläche des Magazinkörpers im wesentlichen dicht geschlossen ist, so daß hier ein an die Anschlußöffnung angeschlossener, ringförmiger Druckraum gebildet ist, aus welchem eventuell einströmende Luft nur durch das innere der Kapsel entweichen kann.

Diese Luft strömt durch den von der Stechnadel erzeugten Längskanal des Mediums, wobei sie das Medium von dessen Wandung unten Verwirbelung abträgt und mitreißt, so daß es bereits am Auslaß der Kapsel fein und gleichmäßig verwirbelt ausgetragen wird. Die Austragöffnung kann auch dafür vorgesehen sein, zum Gebrauch der Austragvorrichtung einen Austragstutzen gegen die Öffnungseinrichtung austauschbar anzuordnen.

Die Austragvorrichtung kann ferner zur Aufnahme nur einer einzigen Einzeldosis bzw. Kapsel des Mediums vorgesehen und nach jedem Gebrauch von außen neu zu füllen sein. Sie ist dabei im wesentlichen über ihre gesamte Länge symmetrisch zur Zentralachse ausgebildet, wobei die erste Handhabe jedoch in einer Querrichtung über den Außenumfang des Gehäuses beiderseits weiter vorsteht als in der dazu rechtwinkligen Querrichtung. Der Grundkörper wird im Bereich des Gehäuseraumes am Außenumfang von einer Steckhülse des Deckels zerstörungsfrei lösbar übergriffen, wobei der Austragstutzen in diesem Fall ein Bauteil mit dem Deckel bildet und im Zentrum über die Außenseite bzw. Druckfläche der ersten Handhabe frei vorsteht.

Die einzige Magazin- bzw. Vorlagekammer kann durch zwei in ihrer Längsrichtung aneinander schließende, hülsenförmige Kammerteile die in Austragstellung mit ihren einander zugekehrten Endflächen im wesentlichen dicht aneinanderliegend oder auch im Abstand voneinander vorgesehen sein können, da die eingesetzte Kapsel eine eventuelle Lücke zwischen den Kammerteilen als Dichtmantel überbrücken kann. Der erste Kammerteil ist unter Zwischenlage des Öffnungswerkzeuges der Öffnungseinrichtung als gesonderter Baukörper in eine Halterungsöffnung des Deckels bzw. des Austragstutzens eingesetzt, und diese Halterungsöffnung kann in einfacher Weise durch den unmittelbar vor der Düse liegenden Innenumfang des Förderweges gebildet sein.

Der erste Kammerteil steht mit seinem offenen Ende frei in den Gehäuseraum 14k und über die Innenseite einer Stirnwand des Deckels vor, die etwa in der Ebene der ersten Handhabe liegt und über welche in gleicher Richtung auch die Steckhülse vorsteht. Der zweite Kammerteil steht in entgegengesetzter Richtung frei über die Querwand vor, mit welcher er einteilig ausgebildet sein kann, so daß die Anschlußöffnung in diesem Fall etwa in der Querwand und unmittelbar am zugehörigen Ende des Druckraumes liegt. Die eingelegte Kapsel grenzt daher mit ihrem einlaßseitigen Ende unmittelbar an den Druckraum an.

Das Öffnungswerkzeug der Öffnungseinrichtung kann im wesentlichen innerhalb des Druckraumes liegen, gegen dessen zugehörige Endfläche es gegen die Kraft einer Feder abhebbar angelegt ist, so daß der Stechdorn in die Anschlußöffnung hineinragt. Zur Federbelastung des Öffnungswerkzeuges kann die Rückstellfeder vorgesehen sein, so daß wegabhängig bzw. mit zunehmendem Betätigungshub die Federkraft ansteigt.

Zum Laden der Austragvorrichtung wird der Deckel vom Grundkörper abgezogen und eine Kapsel in den zweiten Kammerteil so eingesetzt, daß sie über dessen freies Ende vorsteht und mit ihrer Endfläche am Stechdorn anliegt. Danach wird der Deckel so aufgesetzt, daß der erste Kammerteil das vorstehende Ende der Kapsel aufnimmt, wobei dieses Ende im ersten Kammerteil anschlägt, so daß die Kapsel dann in ihre Anschlaglage gegenüber dem zweiten Kammerteil mitgenommen wird. Hierbei kann die Kapsel das Öffnungswerkzeug entgegen der Kraft der Feder mitnehmen, so daß sie zunächst noch nicht oder nur unvollständig geöffnet ist. Mit Beginn des Betätigungshubes der Luftpumpe steigt die in Öffnungsrichtung auf das Öffnungswerkzeug wirkende Federkraft, bis der Stechdorn unter dieser Kraft in die Kapsel eindringt, diese öffnet und dadurch der Einlaß gebildet ist, welcher dann nach Art eines durch Zerstörung zu öffnenden Ventiles den Förderweg freigibt. Das Öffnungswerkzeug der Öffnungseinrichtung kann starr gelagert sein, so daß es bereits beim Aufsetzen des Deckels bzw. vor Öffnung des Einlasses zur Öffnung des Auslasses der Kapsel führt.

Der Endkanal, in welchen die Düse 78 gemäß Fig. 11 mündet, kann um wesentlich mehr als seine geringste oder größte Wandungsdicke enger als der Außenumfang des Austragsstutzens 60 sein, der annähernd flachoval bzw. flach rautenförmige Außenquerschnitte mit einer geringsten Weite aufweisen kann, die der Außenweite des den Endkanal 55 begrenzenden Rohrabschnittes entspricht. Dieser, einteilig mit dem übrigen Austragstutzen ausgebildete Rohrabschnitt schließt dann an zwei voneinander abgekehrten Seiten an Blindräume an, die von dem Austragstutzen begrenzt und etwa in einer Ebene mit der Austragöffnung offen, jedoch ansonsten geschlossen sind. Dadurch ist der Austragstutzen trotz engerer, im wesentlichen zylindrischer Ausbildung des Endkanales, als Mundstück gut geeignet, in den Mund eingeführt und zwischen den Lippen am Außenumfang abgedichtet aufgenommen zu werden.

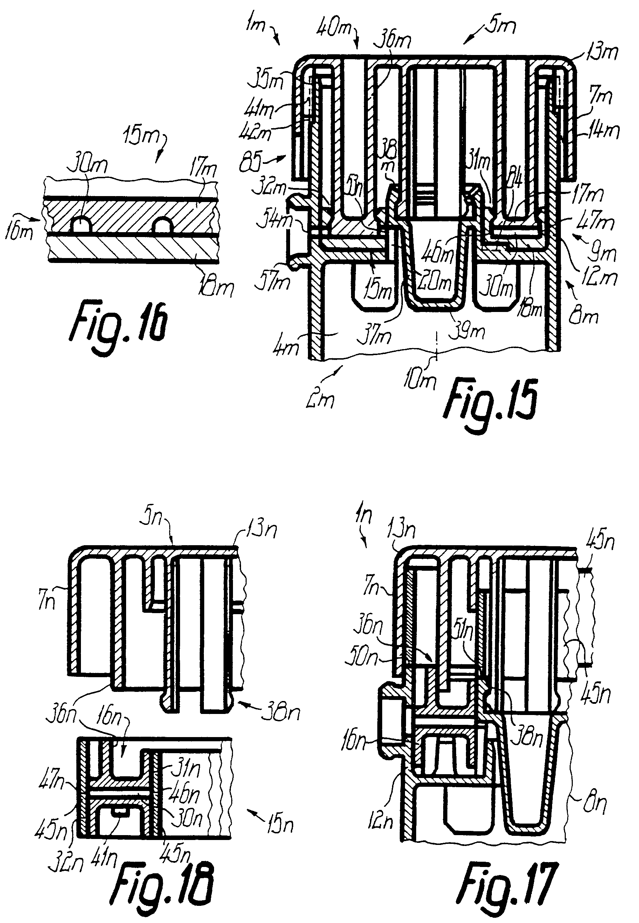

Die Austragvorrichtung 1m nach den Fig. 15 und 16 weist einen Magazinkörper 16m auf, bei welchem für die Vorlagekammern 30m Radialnuten nur in der Stirnfläche des einen Bauteiles 17m vorgesehen sind, während das andere Bauteil 18m eine diese Nuten an ihren offenen Längsseiten verschließende Stirn- bzw. Gleitfläche aufweist. Dieses ringförmige Bauteil 18m ist im Querschnitt durch den jeweiligen Ringabschnitt U-förmig, umgreift mit seinen U-Schenkeln das anderen Bauteil 17m am Innenumfang und Außenumfang und ist mit diesem Bauteil 17m über axial zusammengesetzte Schnappverbindungen zu einer Baueinheit verbunden, die die Radiallager 31m, 32m wie auch die Axiallager bildet. Das Bauteil 17m ist fest bzw. einteilig mit der Kappe 13m verbunden. Zu diesem Zweck weist die Kappe 13m von der Innenseite ihrer Stirnwand vorstehende Stützvorsprünge 36m auf, welche an ihren freien Enden einen das Bauteil 17m bildenden, ringscheibenförmigen Endteil aufweisen. Dieser Endteil weist am Innen- und Außenumfang jeweils einen über die Stützvorsprünge 36m vorstehenden Ringbund auf, der ein Schnappglied der zugehörigen Schnappverbindung 31m, 32m bildet. Der gesamte Magazinkörper 16m einschließlich des Bauteiles 18m bildet so eine in sich geschlossen vormontierte Baueinheit mit den Handhaben 5m, 7m, die zum Auswechseln des Magazines 15m als Ganzes vom Grundkörper 8m entfernt wird. Nach Abziehen des ringdeckelförmigen Bauteiles 18m unter Entrastung der Schnappverbindungen sind die Vorlagekammern 30m über ihre gesamte Länge offen und sie können daher wieder gefüllt und durch Aufschnappen des Deckels 18m geschlossen werden.

Das Bauteil 18m verschließt in diesem Fall auch die Einlässe 46m und Auslässe 47m aller derjenigen Vorlagekammern 30m, die nicht in Austragposition stehen, mit seinem inneren bzw. äußeren U-Schenkel. Diese Schenkel sind von kurzen, mit den Anschlußöffnungen 53n 54n fluchtenden Zwischenkanälen nur in dem Bereich durchsetzt, in welchem eine Vorlagekammer 30m in Austragstellung steht. Das Bauteil 17m kann mit der Handhabe 7m gleitend gegenüber dem Bauteil 18m verdreht werden, welches gegenüber dem Grundkörper 8m durch eine axial ein- und ausrückende Verdrehsicherung lagegesichert sowie gegenüber den Anschlußöffnungen 53n, 54n genau ausgerichtet ist. Beim Drehen läuft die von den Vorlagekammern 30m durchsetzte Stirnfläche des Bauteiles 17m so auf der inneren ebenen Stirnfläche des Bauteiles 18m, daß die Vorlagekammern 30m an ihrer Nutlängsseite stets dicht geschlossen bleiben. Entsprechend bleiben auch die Einlässe 46m, 47m durch die inneren Umfangsflächen des Bauteiles 18m dicht geschlossen. Die Magazinkammern 30m durchsetzen somit nur den Außen- und Innenumfang des Bauteiles 17m und nicht das Bauteil 18m. Zur besseren Abdichtung können die aneinander gleitenden Flächen mit Dicht- und Gleitbelägen beschichtet sein. Der beschriebene Planverschluß ermöglicht eine sehr einfache Befüllung der Vorlagekammern 30m, und außerdem werden beim Auswechseln des Magazines 15m die Dicht- und/oder Lagerflächen ebenfalls stets erneuert, so daß Abnutzungen in Grenzen gehalten werden.

Der die Handhabe 7m bildende Mantel des Deckels 13m übergreift hier den Gehäuseteil 12m am Außenumfang. Die Rastung 35m liegt zwischen dem Innenumfang des Deckels 13m und dem Außenumfang des Gehäuseteiles 12m, wobei die federnden Rastglieder 41m in Form langgestreckter Rastzungen einteilig mit dem Deckel 13m ausgebildet sind und in eine Zahnung am Außenumfang des Gehäuseteiles 12m eingreifen. Die Rastglieder gelangen wie die Verdrehsicherung des Bauteiles 18m beim Abziehen des Deckels 13m von selbst außer Eingriff und beim Einsetzen des Deckels von selbst in Eingriff. Der Innenkörper 37m ist hier einteilig mit dem Grundkörper 8m ausgebildet.

Die Austragvorrichtung 1n gemäß den Fig. 17 und 18 weist ein Magazin 15n auf, bei dem vor dem Einsetzen in die Austragvorrichtung die Einlässe 46n und die Auslässe 47n aller Vorlagekammern 30n durch zwei koaxiale, hülsenförmige Verschlußglieder 45n, 47n jeweils gemeinsam verschlossen sind. Das äußere, in sich formstabile und durchgehend konstante Außen- und/oder Innenquerschnitte aufweisende Verschlußglied 45n reicht über die gesamte Länge des Magazinkörpers 16n, so daß es nicht nur einen äußeren Schutzmantel, sondern auch einen Schutz für die Stirnflächen des Magazinkörpers 16n bildet. Das innere, entsprechend ausgebildete Verschlußglied 45n reicht nur über die Länge der inneren Nabe des Magazinkörpers 16n, welche gleich lang wie die Gesamtlänge des Magazinkörpers 16n ist, jedoch länger als die äußere Nabe.

Die Öffnungswerkzeuge 50n, 51n für das äußere und das innere Verschlußglied 45n sind in diesem Fall einerseits durch die zugehörige, ringförmige Stirnfläche an dem beim Deckel 13n liegenden Ende des Gehäuseteiles 12n und andererseits durch die Endflächen der Schnappfeder der Schnappverbindung 38n gebildet, die zweckmäßig jeweils gleiche Außen- und/oder Innenweite wie das zugehörige Verschlußglied 45n haben. Zum Einsetzen eines Magazines 15n wird der mit den Verschlußgliedern 45n versehene Magazinkörper 16n axial in die offene Seite des Deckels 13n bis zu einer Anschlagstellung eingesetzt. Dabei bleiben die Verschlußglieder 45n in ihrer Verschlußlage. Wird nun der so zu einer Baueinheit mit dem Magazin 15n verbundene Deckel 13n auf den Grundkörper 8n aufgesetzt, so schlagen die von der Deckelstirnwand abgekehrten Stirnflächen der Verschlußglieder 45n an den Öffnungsgliedern 50n, 51n an, so daß sie bei weiterem Einführen des Magazinkörpers 16n in den Gehäuseteil 12n gegenüber diesem stehen bleiben, vom Magazinkörper 16n axial abgestreift werden und in den Deckel 13n verschoben werden.

Am Ende dieser Bewegung rastet die Schnappverbindung 38n ein, so daß die Betriebslage aller genannten Bauteile gesichert ist. Die Verschlußglieder 45n befinden sich dann aufbewahrt innerhalb des Deckels 13n und können zur Erhöhung von dessen mechanischer Festigkeit beitragen. Beim Verschieben ergeben die Verschlußglieder 45n auch die Einlässe 46n und die Auslässe 47n frei, so daß diese dann durch die zugehörigen inneren Gleitflächen des Gehäuseteiles 12n verschlossen sind. Ist das Magazin 15n geleert und wird es ausgewechselt, so können auch die Verschlußglieder 45n aus dem Deckel 13n leicht herausgezogen werden.

Die Kupplung 36n zur formschlüssigen Drehmitnahme des Magazinkörpers 16n durch die Handhabe 7n ist hier durch zwei teleskopartig ineinander greifende Hülsen gebildet, die eine von der kreiszylindrischen Form abweichende Form haben, nämlich nach Art eines Kreissektors an einer Umfangsstelle komplementär abgeflacht sind. Dadurch kann der Magazinkörper 16n nur in einer einzigen Drehlage mit dem Deckel 13n zusammengesetzt werden, wobei zweckmäßig die von der Innenseite der Stirnwand des Deckels 13n abstehende Mitnehmerhülse am Innenumfang der Gegenhülse des Magazinkörpers 16n liegt. Diese Gegenhülse bildet gleichzeitig die radial äußere Lagernabe des Magazinkörpers 16n. In Betriebslage liegt das äußere Verschlußglied 45n eng am Innenumfang des Mantels des Deckels 13n an und umgibt mit Radialabstand den Außenumfang der Kupplungshülse. Innerhalb dieser kann mit Radialabstand eine weitere Innenhülse des Deckels 13n vorgesehen sein, an welcher in Betriebslage das innere Verschlußglied 45n mit seinem Außenumfang entsprechend eng bzw. reibungsschlüssig verbunden so anliegt, daß es mit seinem Innenumfang die Rastlage der dem Deckel 13 zugehörigen Rastglieder der Schnappverbindung 38n nicht behindert.

Die Austragvorrichtung kann auch mit einer Anzeigeeinrichtung 85 gemäß Fig. 15 versehen sein, mit welcher beliebige Daten erfasst, gespeichert und angezeigt werden können, z.B. die Anzahl der entleerten oder noch gefüllten Magazinkammern 30m. Die Anzeigeeinrichtung 85 kann in einfacher Weise durch ein vor einer Skala laufendes Fenster zwischen zwei gegeneinander verdrehbaren Bauteilen gebildet sein, z.B. durch ein Fenster im Mantel des Deckels 13m und eine Skala am Außenumfang des Gehäuseteiles 12m, wobei das Anzeigefenster jeweils nur eine einzige Skaleneinheit von der Außenseite der Austragvorrichtung 1m her sichtbar frei gibt.

Die Merkmale der Ausführungsformen sind auch der Übersichtlichkeit halber in ihrer jeweiligen Kombination anhand der einzelnen Ausführungsbeispiele dargestellt oder beschrieben. Diese Merkmale können jedoch in beliebiger Kombination und/oder Addition an weiteren, erfindungsgemäßen Ausführungsformen vorgesehen sein. Insbesondere die Merkmale der Grundkörper und Gehäuse sowie der Handhaben, der Magazine bzw. Magazinkörper, der Förderwege, der Öffnungseinrichtungen, der Endkanalausbildung, der Schalttriebe, der Steuermittel und der Nachfülleinrichtungen können in dieser Weise kombiniert werden. Auch können zwei oder mehr gleiche und/oder verschiedene Austragvorrichtungen 1, 1a, 1c, 1f, 1m, 1n zu einer Baueinheit zusammengefaßt werden, z.B. um wahlweise unterschiedliche Medien gleichzeitig und/oder aufeinanderfolgend ausbringen zu können.

## Patentansprüche

1. Austragvorrichtung für fließfähige Medien, insbesondere pulverförmige Medien, mit einer Austragbetätigung (3), einem Austragförderer (2), einem Magazin für das Medium und einer Austragoffnung (11), wobei an einem Grundkörper (8) in einem Austragzustand als Kanalabschnitt eines Förderweges (20) mindestens eine Vorlagekammer (30) für das Medium zum Ausschieben des Mediums angeschlossen ist, welche mit einer eine Zentralachse (10) definierenden Lagerung (31 bis 34) an dem Grundkörper (8) angeordnet ist, dadurch gekennzeichnet, daß mindestens eine Vorlagekammer (30) quer zur Zentralachse (10) verlaufend angeordnet ist.

2. Austragvorrichtung nach Anspruch 1, dadurch gekennzeichnet, daß das Magazin (15) mindestens einen Magazinkörper (16) mit einer Mehrzahl von gesonderten Magazinkammern (30) aufweist, die zur Bildung jeweils einer Vorlagekammer durch eine Drehbewegung und/oder eine etwa lineare Schiebebewegung aufeinanderfolgend in eine Austragstellung in den Förderweg (20) bewegbar und insbesondere in dieser Austragstellung zur Bildung eines Zwischenabschnittes mit voneinander abgekehrten Enden (46, 47) an anschließende Abschnitte des zur Austragöffnung (11) führenden Förderweges (20) angeschlossen sind, wobei vorzugsweise mindestens eine Magazinkammer (30) zur unmittelbaren Aufnahme des Mediums und/oder mindestens eine Magazinkammer zur Aufnahme einer mit Medium gefüllten und geschlossenen Kapsel (80) ausgebildet ist.

3. Austragvorrichtung nach Anspruch 1 oder 2, dadurch gekennzeichnet, daß mindestens ein Magazinkörper (16) für gesonderte Einzeldosen des Mediums zwischen zwei Betätigungshandhaben (5, 6), insbesondere im Betriebszustand, in mindestens einer von mehreren Lagen angeordnet ist, die durch eine vollständig umkleidete Lage, eine Lage zwischen zwei Axiallagern (33, 34), eine Lage zwischen einem inneren und einem äußeren Radiallager (31, 32), eine axial schrittweise einstellbare Lage, eine Lage axial benachbart zu einer Rückstellfeder (27) für die Austragbetätigung (3), eine Lage um einen Gaskanal, eine Lage unmittelbar benachbart zu einem Druckgasspeicher (21) oder eine Lage in einem durch eine einzige Querwand (22) von einer Pumpkammer getrennten Gehäuseraum (14) definiert sind, wobei vorzugsweise mindestens eine Magazinkammer (30, 30c) kanalförmig radial zur Zentralachse (10, 10c) der Vorrichtung (1, 1c) und/oder mindestens eine Magazinkammer (30d, 30h) etwa achsparallel zu dieser Zentralachse (10d, 10h) angeordnet ist sowie durch Rastung (35, 35b) festsetzbare Schaltstellungen vorgesehen sind, in denen jeweils nur eine Magazinkammer (30, 30b) an die Austragöffnung (11, 11b) bzw. den Druckgasspeicher (21) ventilfrei angeschlossen ist.

4. Austragvorrichtung nach einem der vorhergehenden Ansprüche, dadurch gekennzeichnet, daß mindestens ein Magazinkörper (16, 16a) mit wenigstens einem als Freilauf (35, 36a) ausgebildeten Schaltwerk (40, 40a) in einer Schaltbewegung schrittweise von einer zur nächsten Austragstellung schaltbar ist, das insbesondere mindestens eine von mehreren Schaltbetätigungen aufweist, die durch eine in Richtung der Schaltbewegung im wesentlichen formschlüssige Verbindung mit einer Schalt-Handhabe (7), einen eine Betätigungsbewegung der Austragbetätigung (3a) selbsttätig in die quer dazu liegende Schaltbewegung umsetzenden Schalttrieb und eine die jeweilige Austragstellung federnd durch Rastung arretierende Schaltbetätigung gebildet sind, wobei vorzugsweise das Schaltwerk (40, 40a) an einer Seite und die Rastung an einer davon abgekehrten Seite am Magazinkörper (16, 16a) angreift.

5. Austragvorrichtung nach einem der vorhergehenden Ansprüche, dadurch gekennzeichnet, daß mindestens ein Magazinkörper (16) als ganzes zerstörungsfrei auswechselbar gelagert ist und insbesondere mindestens eine von mehreren Wechsellagerungen aufweist, die durch eine über eine Kupplung (36) lösbare Verbindung mit einer Handhabe (5, 7), eine von einem abnehmbaren Gehäusedeckel (13) abgedeckte Lagerung, eine Stecklagerung, eine axial von einem Dorn (37) abziehbare Lagerung, eine Schnapplagerung (38) oder eine Lagerung mit voneinander trennbaren Rastgliedern (41, 42) einer Schritt-Rastung (35) gebildet sind, wobei der Magazinkörper (16) vorzugsweise über seine gesamte Länge ringförmig ausgebildet und/oder aus mindestens zwei im wesentlichen in einer Mittelebene der Magazinkammern (30) gefügten Bauteilen (17, 18) zusammengesetzt ist.

6. Austragvorrichtung nach einem der vorhergehenden Ansprüche, dadurch gekennzeichnet, daß mindestens eine Vorlagekammer (30m) von wenigstens zwei mindestens über ihre Länge reichenden Bauteilen (17m, 18m) begrenzt ist, die zur Öffnung der Vorlagekammer (30m) gegeneinander bewegbar sind und/oder daß wenigstens ein Magazinkörper (16n) mit mindestens einem Verschlußkörper (45n) für die Enden (46n, 47n) der Magazinkammern (30n) versehen ist, wobei vorzugsweise Mittel (50n, 51n) vorgesehen sind, um den jeweiligen Verschlußkörper (45n) beim Einsetzen des Magazinkörpers (16n) in die Austragvorrichtung (1n) in eine Öffnungslage zu verschieben bzw. in einer Gehäusekappe (13n) aufzunehmen.

7. Austragvorrichtung nach einem der vorhergehenden Ansprüche, dadurch gekennzeichnet, daß mindestens eine Vorlage- bzw. Magazinkammer (30 bzw. 30f) an wenigstens einem Ende mindestens teilweise mit einem Verschlußglied (45 bzw. 80) verschlossen ist, für das eine Öffnungseinrichtung (50, 51 bzw. 50f, 51f) vorgesehen ist, welche insbesondere mindestens eine von mehreren Öffnungsfunktionen aufweist, die durch ein mit dem Anschluß der Vorlagekammer (30, 30f) an die Austragöffnung (11, 11f) zusammenfallendes Öffnen, ein Öffnen durch eine Magazin-Schaltbewegung, ein Öffnen zwischen zwei Öffnungswerkzeugen (48, 49), ein kontinuierlich schneidendes Öffnen, ein Sprengöffnen, ein Reißöffnen, ein Öffnen unter Durchstechen im wesentlichen der gesamten Medienkammer oder ein Öffnen durch ein den Förderweg (20, 20f) mindestens an einer Seite begrenzendes Öffnungswerkzeug (48, 48f) definiert sind, wobei mindestens ein Verschlußglied (45, 80) vorzugsweise wenigstens eine von mehreren Ausbildungen aufweist, die durch eine Ausbildung als haftend befestigte Folie, eine gemeinsame Ausbildung für mindestens zwei bis alle Magazinkammern (30), eine Ausbildung als Banderole oder eine Ausbildung als Kapsel definiert sind.

8. Austragvorrichtung nach einem der vorhergehenden Ansprüche, dadurch gekennzeichnet, daß ein gemeinsames Außenmantel-Gehäuse (9) einen Aufnahmeraum (14) für mindestens eine Vorlagekammer (30) und unmittelbar einen Druckraum (21) eines Generators (4) für einen Förderstrom, wie z.B. einer Luftpumpe (19), bildet, sowie insbesondere wenigstens eine von mehreren Ausbildungen aufweist, die durch einen hülsenförmigen Gehäuse-Außenkörper (12) mit einer im Abstand von beiden Hülsenenden liegenden Querwand (22), einen Innenkörper (37) zur Lagerung des jeweiligen Magazinkörpers (16), einen End-Deckel (13) zum lösbaren Verschluß des Aufnahmeraumes (14) an einem Ende, einen vom anderen Ende her in das Gehäuse eingesetzten Pumpkolben (25) mit Handhabe (6) sowie Halterungen für mindestens ein Öffnungswerkzeug (48, 49, 52) gebildet sind, wobei der Außenmantel des Gehäuses (9) vorzugsweise von höchstens drei durch Schnappverbindungen (26, 38) oder dgl. in Längsrichtung aneinandergesetzten Bauteilen (12, 13, 24) gebildet ist und/oder einen Kupplungsansatz (57) zur wahlweisen Verbindung mit einem Austragstutzen (60) von der Außenseite des Gehäuses (9) her aufweist und wobei ferner insbesondere ein manuell zu betätigender Generator (4) für den Förderstrom etwa achsparallel sowie axial unmittelbar benachbart zu einem Magazin (1) für das Medium liegt.

9. Austragvorrichtung nach einem der vorhergehenden Ansprüche, dadurch gekennzeichnet, daß mindestens eine Vorlagekammer (30a) an einem Einlaß (46a) für einen Druckgas-Förderstrom und/oder an einem Auslaß (47a) für das Medium in Ausgangslage der Austragbetätigung (3a) gegenüber dem Förderweg (20a) geschlossen und wegabhängig von der Austragbetätigung (3a) über Steuermittel geöffnet werden kann, die insbesondere mindestens eine von mehreren Ausbildungen aufweisen, welche durch eine lineare Schiebersteuerung, eine drehende Schiebersteuerung oder eine wegabhängige Zwangssteuerung eines im Förderweg (20a) liegenden Ventiles (62) gebildet sind, wobei der Magazinkörper (16a) vorzugsweise mit einem Steuermantel (65) zwischen einen inneren Mantel des Generators (4a) und einen äußeren Mantel einer Betätigungshandhabe (5a) eingreift und/oder von einer Kolbenstange (37a) der Luftpumpe (19a) verschiebbar durchsetzt ist, die einen gegenüber dem Magazinkörper (16a) freiliegenden sowie von dem Förderweg (20a) durchsetzten Pumpkolben (25a) aufweist, der in wenigstens einer Stellung innerhalb des Steuermantels (65) liegt.

10. Austragvorrichtung nach einem der vorhergehenden Ansprüche, dadurch gekennzeichnet, daß mindestens ein Magazinkörper exzentrisch zu der Zentralachse der Vorrichtung angeordnet ist und insbesondere wenigstens eine von mehreren Ausbildungen aufweist, die durch eine Lagerung mit einem exzentrischen Lagerzapfen, eine in ein Fenster eines Außenmantel-Gehäuses eingreifende Anordnung, eine Scheibenform oder eine freiliegende manuelle Zugänglichkeit von außen gebildet sind, wobei vorzugsweise auf ein Ende des Gehäuses ein Kammeröffner mit einer an einem Steckkörper federbelastet gelagerten Stecknadel aufsetzbar und/oder im Bereich des anderen Endes des Gehäuses eine beiderseits vorstehende Handhabe vorgesehen ist, über die axial ein Stößel mit einer Betätigungshandhabe für den Generator verschiebbar vorsteht.

11. Austragvorrichtung nach einem der vorhergehenden Ansprüche, dadurch gekennzeichnet, daß für mindestens eine Vorlage- bzw. Magazinkammer (30d) eine Nachfülleinrichtung zur Nachfüllung aus einem eine Vielzahl von Einzeldosen des Mediums aufnehmenden Speichermagazin (69) vorgesehen ist, die insbesondere wenigstens eine von mehreren Ausbildungen aufweist, welche durch einen Magazinkörper (16d) mit mindestens einer an zwei gegenüberliegenden Dichtflächen zwischen einer Übertrittsöffnung (71) des Speichermagazins (69) und dem Förderweg (20d) geschlossen bewegbaren Magazinkammer (30d), eine Kolbenbelastung (72) des im Speichermagazin (69) enthaltenen Mediums, eine Verengung der Übertrittsöffnung (71) gegenüber dem Speichermagazin (69), eine Verengung der Übertrittsöffnung (71) gegenüber der Magazinkammer (30d), eine im wesentlichen vollständig eingeschlossene Anordnung des Magazinkörpers (16d), eine zur Austragöffnung (11d) etwa parallele Anordnung des Speichermagazines (69), eine zum Generator (4d) etwa parallele Anordnung des Speichermagazines (69) oder eine im wesentlichen scheibenförmige Ausbildung des Außen-Gehäuses (9d) der Vorrichtung (1d) gebildet sind, wobei der Magazinkörper (16d) vorzugsweise drehbar gelagert ist und auf einem Teil seines Umfanges zur Bildung einer Dreh-Handhabe (7d) freiliegt.

12. Austragvorrichtung nach einem der vorhergehenden Ansprüche, dadurch gekennzeichnet, daß zur Überführung des Mediums von einem eine Vielzahl von Einzeldosen aufnehmenden Speichermagazin zur Vorlagekammer ein durch eine Übertrittsöffnung in einer Trennwand zwischen dem Speichermagazin und der Vorlagekammer bewegbarer Medienschieber vorgesehen ist, der insbesondere wenigstens eine von mehreren Ausbildungen aufweist, die durch eine etwa lineare Verschiebbarkeit, eine Verschiebbarkeit quer zum Förderweg, eine das Speichermagazin quer durchsetzende Führung, eine löffelartige Ausbildung, eine Ausbildung als in die Vorlagekammer einführbarer Vorlageboden, eine Ausbildung als kurvengesteuerter Schieber, eine dornförmige Ausbildung, eine Führung an einem einen Pumpkolben tragenden Grundkörper, eine Führung an einem eine Rüttelvorrichtung für das gespeicherte Medium tragenden Gehäuseteil oder eine über einen Gehäuseteil mit einem Betätigungsabschnitt frei vorstehende Lagerung gebildet sind, wobei vorzugsweise zur wegabhängigen Betätigung des Medienschiebers zwei Gehäuseteile eines Außenmantel-Gehäuses manuell gegeneinander verschiebbar sind und/oder eine Steuerkurve für den Medienschieber einteilig in eine Kolbenlaufbann für die Luftpumpe übergeht.

13. Austragvorrichtung nach einem der vorhergehenden Ansprüche, dadurch gekennzeichnet, daß mindestens eine Vorlage- bzw. Magazinkammer durch ein quer zu ihrer Längsrichtung zwischen den Enden geteiltes, zur Füllung der Vorlagekammer zerstörungsfrei zu öffnendes Kammergehäuse gebildet ist, das insbesondere mindestens eine von mehreren Ausbildungen aufweist, die durch eine zum Generator etwa achsgleiche Lage, eine Baugruppen-Verbindung eines Kammerteiles mit einem abnehmbaren Gehäuseteil eines Außenmantel-Gehäuses, eine einteilige Verbindung eines Kammerteiles mit einem Gehäuseteil des Generators, eine Anordnung eines Kammerteiles an einem die Austragöffnung und eine Betätigungshandhabe aufweisenden Gehäusedeckel, eine an beiden Enden verengte Ausbildung des Kammerraumes oder eine Lage zwischen zwei Öffnungseinrichtungen zum Öffnen eines Verschlusses der Magazinkammer gebildet sind, wobei vorzugsweise ein Öffnungswerkzeug gegen Federkraft bewegbar gelagert und/oder mit einer Rückstellfeder der Austragbetätigung gegen eine angeschlagene Arbeitslage belastet ist.

## Claims

1. Discharge apparatus for flowable media, particularly pulverulent medea, with a discharge actuating means (3), a discharge conveyor (2), a magazine for the medium and a discharge opening (11), in which to a basic body (8) in a discharge state is connected as a channel portion of a feed path (20) at least one reception chamber (30) far the medium for the expulsion of said medium and which is placed with a bearing (31 to 34), defining a central axis (10), on a basic body (8), characterized in that at least one reception chamber (30) is positioned transversely to the central axis (10).

2. Discharge apparatus according to claim 1, characterized in that the magazine (15) has at least one magazine body (16) with a plurality of separate magazine chambers (30) which, for forming in each case one reception chamber are movable by a rotary movement and/or an approximately linear sliding movement successively into a discharge position in the feed path (20) and in particular in said discharge position, for forming an intermediate portion with remote ends (46, 47) are connected to connecting portions of the feed patch (20) leading to the discharge opening (11) and preferably at least one magazine chamber (30) is constructed for the direct reception of the medium and/or at least one magazine chamber is constructed for receiving a capsule (80) filled with medium and closed.

3. Discharge apparatus according to claim 1 or 2, characterized in that at least one magazine body (16) for separate individual doses of the medium is positioned between two operating handles (5, 6), particularly in the operating state, in at least one of several positions, which are defined by a completely enveloped position, a position between two axial bearings (33, 34), a position between an inner and an outer radial bearing (31, 32), an axially stepwise adjustable position, a position axially adjacent to a restoring spring (27) for the discharge actuation (3), a position around a gas duct, a position directly adjacent to a pressurized gas reservoir (21) or a position in a casing chamber (14) separated by a single partition (22) from a pump chamber and preferably at least one magazine chamber (30, 30c) in channel-like manner is positioned radially to the central axis (10, 10c) of the apparatus (1, 1c) and/or at least one magazine chamber (30d, 30h) is approximately axially parallel to said central axis (10d, 10h) and indexing positions fixable by a catch (35, 35b) are provided in which valve-free connection takes place of only one magazine chamber (30, 30b) to the discharge opening (11, 11b) or the pressurized gas reservoir (21).

4. Discharge apparatus according to one of the preceding claims, characterized in that at least one magazine body (16, 16a) with at least one indexing mechanism (40, 40a) constructed as a freewheeling means (35, 36a) is indexable in an indexing movement stepwise from one to the next discharge position, which in particular has at least one of several indexing actuators, which are formed by a positive connection in the direction of the indexing movement with an indexing handle (7), an indexing drive transforming an actuating movement of the discharge actuator (3a) independently into the indexing movement transversely thereto and an indexing actuator arresting the discharge position resiliently by a catch and preferably the indexing mechanism (40, 40a) engages on one side and the catch on a side remote therefrom on the magazine body (16, 16a).

5. Discharge apparatus according to one of the preceding claims, characterized in that at least one magazine body (16) is entirely non-destructively and interchangeably mounted and in particular has at least one of several change bearings formed by a connection detachable by means of a coupling (36) to a handle (5, 7), a bearing covered by a removable casing cover (13), a plug-in bearing, a bearing axially removable from a mandrel (37), a snap bearing (38) or a bearing with separable catch members (41, 42) of a step catch (35), the magazine body (16) preferably being annular over its entire length and/or formed by at least two components (17, 18) joined in a median plane of the magazine chambers (30).

6. Discharge apparatus according to one of the preceding claims, characterized in that at least one reception chamber (30m) is bounded by at least two components (17m, 18m) extending over its length and which are movable against one another for opening the reception chamber (30m) and/or that at least one magazine body (16n) is provided with at least one closure body (45n) for the ends (46n, 47n) of the magazine chambers (30n) and preferably means (50n, 51n) are provided in order to displace into an open position or into a casing cap (13n) the closure body (45n) on inserting the magazine body (16n) in the discharge apparatus (1n).

7. Discharge apparatus according to one of the preceding claims, characterized in that at least one reception or magazine chamber (30 or 30f) is closed at at least one end at least partially with a closure member (45 or 80) for which an opening device (50, 51 or 50f, 51f) is provided, which in particular has at least one of several opening functions, which are formed by an opening coinciding with the connection of the reception chamber (30, 30f) to the discharge opening (11, 11f), an opening by a magazine indexing movement, an opening between two opening tools (48, 49), a continuously cutting opening, a spring opening, a tear opening, an opening accompanied by perforation in substantially the entire medium chamber or an opening by an opening tool (48, 48f) bounding the feed path (20, 20f) on at least one side, in which at least one closure member (45, 80) preferably has at least one of several constructions defined by a construction as an adhesively fixed foil, a joint construction for at least two to all the magazine chambers (30), a construction as a banderole or a construction as a capsule.

8. Discharge apparatus according to one of the preceding claims, characterized in that a common outer jacket casing (9) forms a reception space (14) for at least one reception chamber (30) and directly a pressure space (21) of a generator (4) for a feed flow such as e.g. an air pump (19), as well as having in particular at least one of several constructions defined by a sleeve-like casing outer body (12) with a partition (22) spaced from both sleeve ends, an inner body (37) for mounting the magazine body (16), an end cover (13) for the detachable closure of the reception space (14) at one end, a pump piston (25) with handle (6) inserted from one end in the casing, as well as mounting supports for at least one opening tool (48, 49, 52), in which the outer jacket of the casing (9) is preferably formed by at the most three components (12, 13, 24) engaged with one another by snap connection (26, 38) or the like in the longitudinal direction and/or a coupling shoulder (57) for the, as desired, connection to a discharge connecting piece (60) from the outside of the casing (9) and in which there is also a manually operable generator (4) for the feed flow approximately axially parallel and axially directly adjacent to a magazine (15) for the medium.

9. Discharge apparatus according to one of the preceding claims, characterized in that at least one reception chamber (30a) is closed at an inlet (46a) for a compressed gas feed flow and/or at an outlet (47a) for the medium in the starting position of the discharge actuator (3a) with respect to the feed path (20a) and can be opened by control means in path-dependent manner by the discharge actuator (3a) and having in particular at least one of several constructions, formed by a linear slide control, a rotary slide control or a path-dependent forced control of a valve (62) located in the feed path (20a), in which the magazine body (16a) preferably engages with a control jacket (65) between an inner jacket of the generator (4a) and an outer jacket of the actuating handle (5a) and/or is displaceably traversed by a piston rod (37a) of the air pump (19a), which has a pump piston (25a) exposed with respect to the magazine body (16a) and traversed by the feed path (20a) and which in at least one position is within the control jacket (65).

10. Discharge apparatus according to one of the preceding claims, characterized in that at least one magazine body is arranged eccentrically to the central axis of the apparatus and in particular has at least one of several constructions formed by a bearing with an eccentric bearing journal, an arrangement engaging in a window of an outer jacket casing, a disk shape or an exposed manual accessibility from the outside and preferably on one end of the casing can be placed a chamber opener with a perforating needle mounted in spring-loaded manner on a perforating body and/or in the vicinity of the other end of the casing is provided a handle projecting on both sides and over which axially displaceably projects a ram with an actuating handle for the generator.

11. Discharge apparatus according to one of the preceding claims, characterized in that for at least one reception or magazine chamber (30d) there is a refilling device for refilling from a storage magazine (69) receiving a plurality of individual medium doses and which in particular has at least one of several constructions formed by a magazine body (16d) with at least one closed movable magazine chamber (30d) at two opposite sealing faces between a transfer opening (71) of the storage magazine (69) and the feed path (20d), a piston loading means (72) of the medium contained in the storage magazine (69), a constriction of the transfer opening (71) with respect to the storage means (69), a constriction of the transfer opening (71) with respect to the magazine chamber (30d), a substantially completely enclosed arrangement of the magazine body (16d), an arrangement of the storage magazine (69) roughly parallel to the discharge opening (11d), an arrangement of the storage magazine (69) roughly parallel to the generator (4d) or a substantially disk-like construction of the outer casing (9d) of the apparatus (1d), the magazine body (16d) preferably being mounted in rotary manner and is exposed on part of its circumference for forming a rotary handle (7d).

12. Discharge apparatus according to one of the preceding claims, characterized in that for the transfer of the medium from a storage magazine receiving a plurality of individual doses to the reception chamber is provided a medium slider movable through a transfer opening in a partition between the storage magazine and the reception chamber and which in particular has at least one of several constructions formed by an approximately linear displaceability, a displaceability transversely to the feed path, a guide transversely traversing the storage magazine, a spoon-like construction, a construction as a reception base insertable into the reception chamber, a construction as a cam-controlled slider, a mandrel-like construction, a guide on a basic body carrying a pump piston, a guide on a casing part carrying a shaking device for the stored medium, or a bearing projecting freely over a casing part with an actuating portion and preferably for the path-dependent actuation of the medium slider two casing parts of an outer jacket casing are manually displaceable against one another and/or a control cam for the medium slider passes in one piece into a piston race for the air pump.

13. Discharge apparatus according to one of the preceding claims, characterized in that at least one reception or magazine chamber is formed by a non-destructively openable chamber casing divided transversely to its longitudinal direction between the ends for the filling of the reception chamber and which in particular has at least one of several constructions, formed by a position roughly equiaxial to the generator, a component connection of a chamber part to a removable casing part of an outer jacket casing, a one-piece connection of a chamber part to a casing part of the generator, an arrangement of a chamber part on a casing cover having a discharge opening and an actuating handle, a construction of the chamber space constricted at both ends or a position between two opening devices for opening a closure of the magazine chamber and preferably an opening tool is movably mounted against spring tension and/or loaded with a restoring spring of the discharge actuator against a striking working position.

## Revendications

1. Distributeur de fluides, notamment de fluides pulvérulents, avec un actionneur de distribution (3), un refouleur de distribution (2), un magasin pour le fluide et une ouverture de distribution (11), au moins une chambre (30) de préparation du fluide en vue de son expulsion étant, dans un état de distribution, raccordée à un corps de base (8) en tant que tronçon de canal d'un parcours de refoulement (20), chambre qui est disposée sur le corps de base (8) par des paliers (31 à 34) qui définissent un axe central (10), **caractérisé** en ce qu'au moins une chambre de préparation (30) est disposée en s'étendant transversalement à l'axe central (10).

2. Distributeur selon la revendication 1, **caractérisé** en ce que le magasin (15) présente au moins un corps de magasin (16) avec une pluralité de chambres de magasin (30) séparées qui, en vue de former des chambres de préparation respectives, peuvent être successivement déplacées, par un mouvement de rotation et/ou par un mouvement de translation approximativement linéaire, dans une position de distribution dans le parcours de refoulement (20), et qui, dans cette position de distribution, afin de former un tronçon intermédiaire, sont notamment raccordées par des extrémités mutuellement opposées (46, 47) à des tronçons attenants du parcours de refoulement (20) menant à l'ouverture de distribution (11), au moins une chambre de magasin (30) étant de préférence conçue pour recevoir directement le fluide, et/ou au moins une chambre de magasin étant conçue pour recevoir une capsule (80) fermée et remplie de fluide.

3. Distributeur selon la revendication 1 ou 2, **caractérisé** en ce qu'au moins un corps de magasin (16) pour des doses individuelles séparées de fluide est disposé entre deux manettes d'actionnement (5, 6), notamment dans l'état de service, dans au moins une de plusieurs positions qui sont définies par une position entièrement enveloppée, une position entre deux paliers axiaux (33, 34), une position entre un palier radial intérieur et un palier radial extérieur (31, 32), une position réglable axialement pas à pas, une position au voisinage axial d'un ressort de rappel (27) pour l'actionneur de distribution (3), une position autour d'un canal de gaz, une position au voisinage immédiat d'un réservoir de gaz comprimé (21) ou une position dans une chambre de boîtier (14) séparée d'une chambre de pompe par une unique cloison transversale (22), au moins une chambre de magasin (30, 30c) étant de préférence disposée en forme de canal radialement à l'axe central (10, 10c) du distributeur (1, 1c) et/ou au moins une chambre de magasin (30d, 30h) étant disposée approximativement en parallélisme axial à cet axe central (10d, 10h), et des positions de commutation pouvant être bloquées par un moyen de crantage (35, 35b) étant prévues, dans chacune desquelles une seule chambre de magasin (30, 30b) est respectivement raccordée sans soupape à l'ouverture de distribution (11, 11b) ou au réservoir de gaz comprimé (21).

4. Distributeur selon une des revendications précédentes, **caractérisé** en ce qu'au moins un corps de magasin (16, 16a) peut être commuté pas à pas, en un mouvement de commutation, d'une position de distribution à la suivante par au moins un dispositif de commutation (40, 40a) réalisé sous forme de roue libre (35, 36a), dispositif qui présente notamment un de plusieurs actionneurs de commutation qui sont formés par une liaison essentiellement positive dans la direction du mouvement de commutation avec une manette de commutation (7), un mécanisme de commutation transformant automatiquement un mouvement d'actionnement de l'actionneur de distribution (3a) en mouvement de commutation transversal audit mouvement, et un actionneur de commutation bloquant par crantage élastique la position de distribution respective, le dispositif de commutation (40, 40a) agissant de préférence sur un côté et le crantage sur le côté opposé du corps de magasin (16, 16a).

5. Distributeur selon une des revendications précédentes, **caractérisé** en ce qu'au moins un corps de magasin (16) est monté en pouvant être remplacé dans sa totalité sans destruction et présente notamment au moins un de plusieurs montages alternatifs, qui sont formés par un assemblage détachable à une manerte (5, 7) au moyen d'un accouplement (36), un montage recouvert par un couvercle de boîtier amovible (13), un montage à emboîtement, un montage pouvant être retiré axialement par un mandrin (37), un montage à enclenchement (38) ou un montage avec des organes de crantage (41, 42), pouvant être mutuellement séparés, d'un moyen de crantage graduel (35), le corps de magasin (16) étant de préférence réalisé de forme annulaire sur toute sa longueur, et/ou étant composé d'au moins deux éléments de construction (17, 18) réunis essentiellement dans un plan médian des chambres de magasin (30).

6. Distributeur selon une des revendications précédentes, **caractérisé** en ce qu'au moins une chambre de préparation (30m) est délimitée par au moins deux éléments de construction (17m, 18m) s'étendant au moins sur sa longueur, qui peuvent être déplacés l'un par rapport à l'autre pour ouvrir la chambre de préparation (30m), et/ou en ce qu'au moins un corps de magasin (16n) est pourvu d'au moins un corps d'obturation (45n) pour les extrémités (46n, 47n) des chambres de magasin (30n), des moyens (50n, 51n) étant de préférence prévus pour, lors de l'insertion du corps de magasin (16n) dans le distributeur (1n), déplacer le corps d'obturation respectif (45n) dans une position d'ouverture, ou encore le recevoir dans un capuchon de boîtier (13n).

7. Distributeur selon une des revendications précédentes, **caractérisé** en ce qu'au moins une chambre de préparation ou chambre de magasin (30 ou 30f) est fermée au moins partiellement, à au moins une extrémité, par un organe d'obturation (45 ou 80) pour lequel est prévu un dispositif d'ouverture (50, 51 ou 50f, 51f) qui présente notamment au moins une de plusieurs fonctions d'ouverture qui sont définies par une ouverture coïncidant avec le raccordement de la chambre de préparation (30, 30f) à l'ouverture de distribution (11, 11f), une ouverture par un mouvement de commutation du magasin, une ouverture entre deux outils d'ouverture (48, 49), une ouverture par découpe continue, une ouverture par éclatement, une ouverture par arrachement, une ouverture en transperçant sensiblement la totalité de la chambre de fluide ou une ouverture par un outil d'ouverture (48, 48f) délimitant sur au moins un côté le parcours de refoulement (20, 20f), au moins un organe d'obturation (45, 80) présentant de préférence au moins une de plusieurs exécutions, qui sont définies par une exécution en feuille fixée par adhérence, une exécution commune pour au moins deux à toutes les chambres de magasin (30), une exécution en bandelette ou une exécution en capsule.

8. Distributeur selon une des revendications précédentes, **caractérisé** en ce qu'un boîtier commun d'enveloppe extérieure (9) forme une chambre réceptrice (14) pour au moins une chambre de préparation (30) et, directement, une chambre de pression (21) d'un générateur (4) de courant de refoulement tel, par exemple, qu'une pompe à air (19), et il présente notamment au moins une de plusieurs configurations, qui sont formées par un corps extérieur de carter (12) en forme de manchon pourvu d'une cloison transversale (22) située à distance des deux extrémités du manchon, un corps intérieur (37) pour le montage du corps de magasin respectif (16), un couvercle termina (13) pour la fermeture amovible de la chambre réceptrice (14) à une extrémité, un piston de pompe (25) inséré par l'autre extrémité dans le boîtier et pourvu d'une manette (6), ainsi que des attaches pour au moins un outil d'ouverture (48, 49, 52), l'enveloppe extérieure du boîtier (9) étant de préférence formée d'au plus trois éléments de construction (12, 13, 24) assemblés bout à bout en direction longitudinale par des assemblages à enclenchement (26, 38) ou analogues, et/ou présentant un appendice d'accouplement (57) pour l'assembler, au choix, à un embout de distribution (60) par le côté extérieur du boîtier (9), et un générateur (4) à actionner manuellement pour le courant de refoulement se trouvant en outre notamment en parallélisme axial ainsi qu'axialement directement voisin d'un magasin (15) pour le fluide.

9. Distributeur selon une des revendications précédentes, **caractérisé** en ce qu'au moins une chambre de préparation (30a) est, dans la position initiale de l'actionneur de distribution (3a), fermée par rapport au parcours de refoulement (20a) à une admission (46a) pour un courant de gaz comprimé de refoulement et/ou à une évacuation (47a) pour le fluide, et peut être ouverte en fonction de la course de l'actionneur de distribution (3a) par des moyens de commande qui présentent notamment au moins une de plusieurs configurations, qui sont formées par une commande à tiroir linéaire, une commande à tiroir rotatif ou une commande forcée en fonction de la course d'une soupape (62) située dans le parcours de refoulement (20a), le corps de magasin (16a) s'engageant de préférence, par une enveloppe de commande (65), entre une enveloppe intérieure du générateur (4a) et une enveloppe extérieure d'une manette d'actionnement (5a) et/ou étant traversé en coulissement par une tige de piston (37a) de la pompe à air (19a), qui présente un piston de pompe (25a) situé à découvert par rapport au corps de magasin (16a) et traversé par le parcours de refoulement (20a), piston qui se trouve, dans au moins une position, à l'intérieur de l'enveloppe de commande (65).

10. Distributeur selon une des revendications précédentes, **caractérisé** en ce qu'au moins un corps de magasin est disposé excentriquement par rapport à l'axe central du distributeur et présente notamment au moins une de plusieurs configurations, qui sont formées par un montage avec un tourillon excentrique, une disposition par engagement dans une fenêtre d'un boîtier d'enveloppe extérieure, une forme de disque ou une possibilité d'accès manuel de l'extérieur à découvert, un moyen pour ouvrir la chambre, pourvu d'une aiguille à enfoncer montée sous sollicitation de ressort sur un corps à enfoncer, pouvant de préférence être monté sur une extrémité du boîtier, et/ou une manette dépassant des deux côtés étant prévue dans la région de l'autre extrémité du boîtier, manette de laquelle dépasse axialement, avec possibilité de translation, un poussoir pourvu d'une manette d'actionnement pour le générateur.

11. Distributeur selon une des revendications précédentes, **caractérisé** en ce qu'un dispositif de remplissage est prévu pour au moins une chambre de préparation ou chambre de magasin (30d), afin de remplir à nouveau cette chambre à partir d'un magasin de stockage (69) recevant une pluralité de doses individuelles de fluide, dispositif qui présente notamment au moins une de plusieurs configurations, qui sont formées par un corps de magasin (16d) pourvu d'au moins une chambre de magasin (30d) pouvant être déplacée pour fermer le passage, au niveau de deux faces d'étanchement en vis-à-vis, entre une ouverture de transfert (71) du magasin de stockage (69) et le parcours de refoulement (20d), une sollicitation par piston (72) du fluide contenu dans le magasin de stockage (69), un étranglement de l'ouverture de transfert (71) par rapport au magasin de stockage (69), un étranglement de l'ouverture de transfert (71) par rapport à la chambre de magasin (30d), une disposition sensiblement totalement enfermée du corps de magasin (16d), une disposition du magasin de stockage (69) approximativement parallèle à l'ouverture de distribution (11d), une disposition du magasin de stockage (69) approximativement parallèle au générateur (4d) ou une configuration essentiellement en forme de disque du boîtier extérieur (9d) du distributeur (1d), le corps de magasin (16d) étant de préférence monté à rotation et dégagé sur une partie de sa surface extérieure pour former une manette rotative (7d).

12. Distributeur selon une des revendications précédentes, **caractérisé** en ce qu'est prévu, afin de transférer le fluide d'un magasin de stockage recevant une pluralité de doses individuelles vers la chambre de préparation, un tiroir pour fluide pouvant être déplacé à travers une ouverture de transfert dans une cloison séparatrice entre le magasin de stockage et la chambre de préparation, tiroir qui présente notamment au moins une de plusieurs configurations, qui sont formées par une possibilité de translation approximativement linéaire, une possibilité de translation transversalement au parcours de refoulement, un guidage traversant transversalement le magasin de stockage, une configuration du genre cuillère, une configuration en fond de préparation pouvant être introduit dans la chambre de préparation, une configuration en tiroir commandé par came, une configuration en forme de mandrin, un guidage sur un corps de base portant un piston de pompe, un guidage sur un élément de boîtier portant un agitateur pour le fluide emmagasiné ou un montage dépassant librement d'un élément de boîtier avec une partie d'actionnement, deux éléments de boîtier d'un boîtier d'enveloppe extérieure pouvant de préférence être déplacés manuellement en translation l'un par rapport à l'autre afin d'actionner le tiroir pour fluide en fonction de la course, et/ou une came de commande pour le tiroir pour fluide se raccordant d'un seul tenant à une voie de déplacement de piston pour la pompe à air.

13. Distributeur selon une des revendications précédentes, **caractérisé** en ce qu'au moins une chambre de préparation ou chambre de magasin est formée par un boîtier de chambre divisé en deux parties transversalement à sa direction longitudinale entre ses extrémités et pouvant être ouvert sans destruction afin de remplir la chambre de préparation, boîtier qui présente notamment au moins une de plusieurs configurations, qui sont formées par une position approximativement de même axe que le générateur, un assemblage en sous-groupe d'une partie de chambre avec une partie démontable de boîtier d'un boîtier d'enveloppe extérieure, un assemblage d'un seul tenant d'une partie de chambre avec une partie de boîtier du générateur, une disposition d'une partie de chambre sur un couvercle de boîtier présentant l'ouverture de distribution et une manette d'actionnement, une configuration rétrécie aux deux extrémités du volume de la chambre ou une position entre deux dispositifs d'ouverture pour ouvrir un obturateur de la chambre de magasin, un outil d'ouverture étant de préférence monté à déplacement à l'encontre d'une force de ressort, et/ou étant sollicité, par un ressort de rappel de l'actionneur de distribution, à l'encontre d'une position de travail en butée.
